(19) Europäisches Patentamt
European Patent Office
Office européen des brevets

(11) Veröffentlichungsnummer: **0 172 526**
**B1**

(12) # EUROPÄISCHE PATENTSCHRIFT

(45) Veröffentlichungstag der Patentschrift: **04.10.89**

(51) Int. Cl.[4]: **C 07 D 471/10**, C 07 D 487/10, A 61 K 31/55

(21) Anmeldenummer: **85110191.5**

(22) Anmeldetag: **14.08.85**

(54) **Neue Benzodiazepine, Verfahren zu ihrer Herstellung sowie ihre Verwendung.**

(30) Priorität: **24.08.84 DE 3431195**

(43) Veröffentlichungstag der Anmeldung:
**26.02.86 Patentblatt 86/9**

(45) Bekanntmachung des Hinweises auf die Patenterteilung:
**04.10.89 Patentblatt 89/40**

(84) Benannte Vertragsstaaten:
**AT BE CH DE FR GB IT LI NL SE**

(56) Entgegenhaltungen:
**EP-A-0 045 452**
**DE-A-2 523 250**
**DE-A-3 422 411**
**US-A-3 998 811**

(73) Patentinhaber: **Troponwerke GmbH & Co. KG, Berliner Strasse 156, D-5000 Köln 80 (DE)**

(72) Erfinder: **Boltze, Karl- Heinz, Dr., Ackerstrasse 8, D-5231 Boron (DE)**
Erfinder: **Etschenberg, Eugen, Dr., Hirseweg 40, D-5000 Koeln 41 (DE)**
Erfinder: **Traber, Jörg, Dr., Löwenburgstrasse 12, D-5204 Lohmar 21 (DE)**
Erfinder: **Büsgen, Herbert, Goethestrasse 36a, D-5216 Niederkassel- Lülsdorf (DE)**

(74) Vertreter: **Mann, Volker, Dr., c/o Bayer AG Konzernverwaltung RP Patentabteilung, D-5090 Leverkusen 1, Bayerwerk (DE)**

**EP 0 172 526 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue Benzodiazepine und deren Säureadditionssalze, Verfahren zu ihrer Herstellung sowie ihre Verwendung bei der Bekämpfung von Krankheiten, insbesondere zur Behandlung altersbedingter cerebraler Störungen und zur Behandlung von Lern- und Gedächtnisstörungen und Amnesien.

Die Erfindung betrifft Benzodiazepine der allgemeinen Formel I:

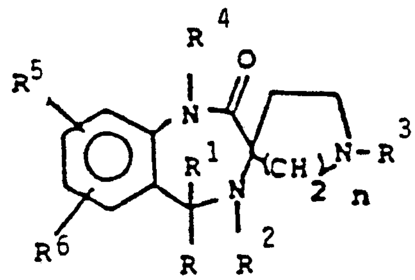

(I)

in welcher

R für H, gesättigtes oder eine Doppelbindung enthaltendes, geradkettiges, verzweigtes oder cyclisches Alkyl mit 1 bis 8 Kohlenstoffatomen, für einen Aralkylrest mit einer Alkyl- oder Alkylenkette von 1 bis 3 C-Atomen und einem unsubstituierten oder gegebenenfalls mit ein oder zwei Substituenten aus der Reihe Cl, Br, F, CN, $CF_3$, $NO_2$, $CH_3$, $C_2H_5$ oder $OCH_3$ versehenen Phenylrest,

für einen gegebenenfalls mit ein oder zwei Substituenten aus der Reihe Cl, F, Br, CN, $CF_3$, $NO_2$, $CH_3$, $C_2H_5$ oder $OCH_3$ versehenen Phenylrest oder einen 5- oder 6-gliedrigen aromatischen Rest mit 1 oder 2 Heteroatomen aus der Reihe O, N, S steht,

$R_1$ für H steht,

$R_2$, $R_3$ und $R_4$ unabhängig voneinander für H, einen Alkylrest mit 1 bis 6 Kohlenstoffatomen, oder für einen Aralkylrest mit einer Alkyl- oder Alkylenkette von 1 bis 3 C-Atomen und einem unsubstituierten oder gegebenenfalls mit einem oder zwei Substituenten aus der Reihe Cl, Br, F, CN, $CF_3$, $NO_2$, $CH_3$, $C_2H_5$ oder $CH_3$ versehenen Phenylrest stehen und gegebenenfalls

wobei

$R_1$ und $R_2$ auch gemeinsam eine Bindung bilden können,

$R_5$ und $R_6$ unabhängig voneinander für H, F, Cl, Br, CN, $CF_3$, $NO_2$, $CH_3$, $C_2H_5$ oder $OCH_3$ stehen und

n für eine ganze Zahl von 1 bis 3 steht.

Die Erfindung bezieht sich, soweit die Verbindungen in Form optischer Antipoden auftreten können, sowohl auf das Racemat als auch auf die einzelnen Enantiomeren. Die Racematspaltung kann auf dem üblichen Weg durch eine Salzbildung mit einer optisch aktiven Säure und eine fraktionierte Kristallisation der diastereomeren Salze erfolgen oder durch eine Chromatographie an einem optisch aktiven Trägermaterial.

Die Benzodiazepine der vorliegenden Erfindung unterscheiden sich von den bekannten Benzodiazepinen eindeutig durch ihre Struktur und ihre pharmakologische Wirkung. Die erfindungsgemäßen Verbindungen haben keine Affinität zu Benzodiazepinrezeptoren des Gehirns und zeigen keine den Benzodiazepinen vergleichbare anxiolytische Wirkung im Tierversuch. Im Gegensatz zu den bekannten Benzodiazepinen haben sie eine starke nootrope Wirkung im Tierversuch. Im Verhaltenstest an gesunden und hirnlädierten Ratten führen sie zu einer deutlichen Verbesserung von Lernfähigkeit und Gedächtnisleistung. Die erfindungsgemäßen Verbindungen eignen sich daher zur Behandlung altersbedingter Störungen der Gehirnfunktion wie den verschiedenen Formen von präseniler und seniler Demenz vom Alzheimer Typ.

Bevorzugt werden Benzodiazepine, bei denen für die Zahl 2 steht.

Besonders bevorzugt sind Verbindungen der Formel I, in denen

R für einen unsubstituierten oder gegebenenfalls mit 1 oder 2 Substituenten aus der Reihe Cl, F, Br, CN, $CF_3$ oder $NO_2$ versehenen Phenylrest, oder einen 5- oder 6-gliedrigen heteroaromatischen Rest mit 1 oder 2 Heteroatomen aus der Reihe O, N, S steht,

$R_1$ für H steht,

$R_2$, $R_3$ und $R_4$ unabhängig voneinander für H oder einen Alkylrest mit 1 bis 4 Kohlenstoffatomen stehen, und wobei

$R_1$ und $R_2$ auch gemeinsam eine Bindung bilden können,

$R_5$ und $R_6$ unabhängig voneinander für H, F, Cl, Br, CN, $CF_3$ oder $NO_2$ stehen und

n für 2 steht.

Weiterhin wurde gefunden, daß man die Verbindungen der Formel I erhält, wenn man Verbindungen der allgemeinen Formel II mit Verbindungen der allgemeinen Formel III zu Verbindungen der allgemeinen Formel

IV cyclisiert

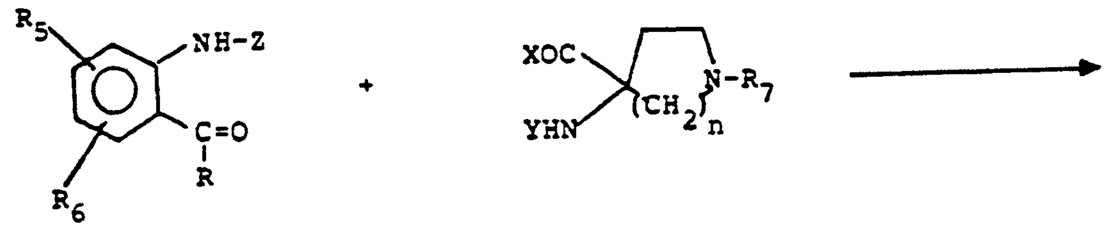

II III

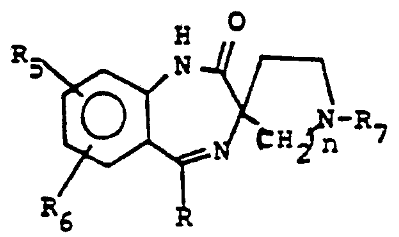

IV

und bei den so erhaltenen Verbindungen der Formel IV für den Fall, daß $R_7$ für eine Schutzgruppe steht, diese abspaltet und gegebenenfalls die Reste $R_2$ und $R_4$ durch Alkylierung einführt.

In den Verbindungen der Formel II und IV haben die Reste $R_5$, $R_6$ und R die oben angegebene Bedeutung, Z steht für H oder eine Schutzgruppe wie beispielsweise Trifluoracetyl.

In den Verbindungen der Formel III steht

COX für eine Carboxylfunktion oder eine aktivierte Carboxylfunktion, wie sie zur Herstellung von Säureamidbindungen Verwendung findet, z. B. für eine Esterfunktion oder eine Carbonsäurechloridfunktion.

Y steht für H oder eine in der Peptidchemie übliche Schutzgruppe, wie z. B. die Carbobenzoxygruppe.

(Lit.: (a) R. B. Merrifield, J. Biol. Chem. 232, 43 (1958);

(b) J.P. Greenstein u. M. Winitz, Chemistry of the Amino Acids, Vol. 2, S. 887-901; John Wiley & Sons, Inc., New York, 1961)

$R_7$ in III und IV hat entweder die oben angegebene Bedeutung von $R_3$ oder steht für eine Schutzgruppe wie beispielsweise Benzyl (T.W. Greene, Protective GROUPS in Organic Synthesis, Seite 272; John Wiley & Sons, Inc., New York, 1981).

Die Cyclisierungsreaktion kann je nach Bedeutung der Reste X, Y und Z entweder in einem Schritt durchgeführt werden, oder aber es kann zuerst die Säureamid-Bindung hergestellt werden und dann gegebenenfalls nach Entfernung der Schutzgruppe Y die C=N-Doppelbindung geknüpft werden,

oder es wird zuerst die C=N-Doppelbindung erzeugt und im zweiten Schritt gegebenenfalls nach Entfernung der Schutzgruppe Z und Aktivierung der Carboxylfunktion die Säureamidbindung hergestellt.

n bedeutet im allgemeinen eine ganze Zahl von 1 bis 3, bevorzugt 2.

Aus Verbindungen der Formel IV lassen sich durch Entfernung der Schutzgruppe $R_7$ Verbindungen der Formel Ia erhalten.

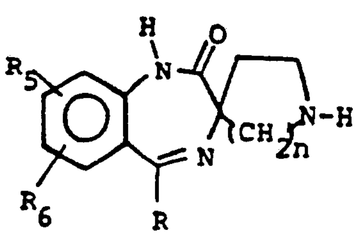

Ia

Die Akylierungsreaktion zur Einführung der Reste $R_2$ und/oder $R_4$ kann selbstverständlich vor oder nach Abspaltung der Schutzgruppe durchgeführt werden.

Aus den Verbindungen der Formel Ia erhält man durch Alkylierung die Verbindungen der Formel Ib ($R_3$ = Alkyl):

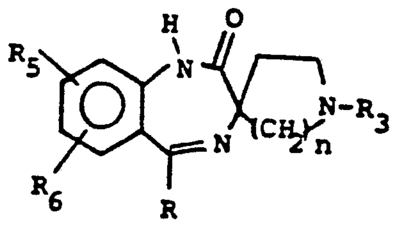

Ib

durch eine zweite Alkylierungsreaktion die Verbindungen Ic ($R_3$ und $R_4$ = Alkyl):

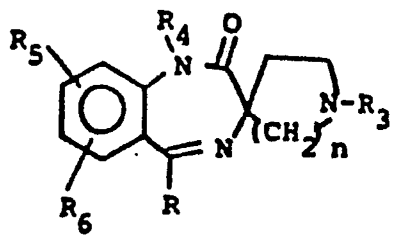

Ic

Die Verbindungen der Formel Id ($R_4$ = Akyl)

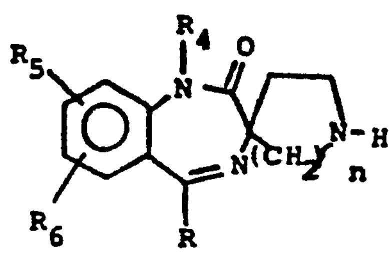

Id

erhält man durch Alkylierung der Verbindungen der Formel IV und anschließende Entfernung der Schutzgruppe $R_7$.

Verbindungen der Formel Ie erhält man durch die Hyrung der Doppelbindung ungesättigter Verbindungen der Formel I.

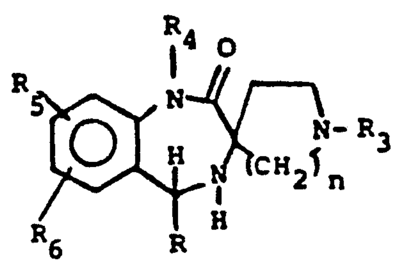

Ie

Die Alkylierung von Verbindungen der Formel Ie ($R_3$ und $R_4$ = Alkyl) liefert Verbindungen der Formel Ig ($R_2$, $R_3$ und $R_4$ = Alkyl):

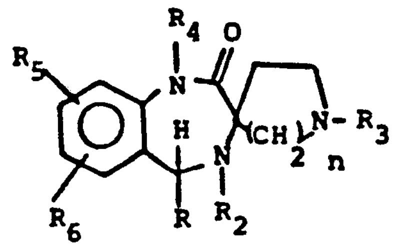

If

Aus Verbindungen der Formel IV lassen sich durch Absättigung der C=N-Doppelbindung mit $H_2$ Verbindungen der Formel V erhalten:

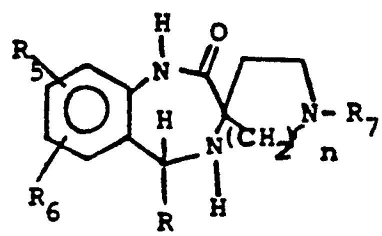

V

Durch ein- oder zweifache Alkylierung der Verbindungen der Formel V und nachfolgende Abspaltung der Schutzgruppe $R_7$ erhält man die Verbindungen Ig ($R_2$ = Alkyl) und Ih ($R_2$ und $R_4$ = Alkyl):

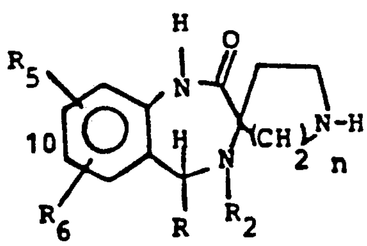

Ig

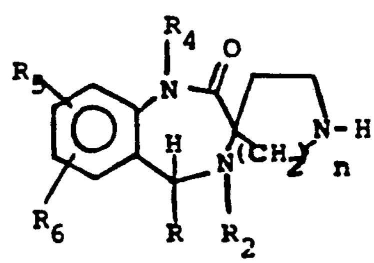

Ih

Schließlich erhält man aus Ig ($R_2$ = Alkyl) durch Alkylierung oder aus Ie ($R_3$, $R_4$ = H) durch zweifache reduktive Alkylierung Verbindungen der Formel Ii ($R_2$ und $R_3$ = Alkyl):

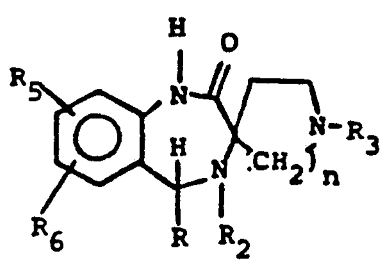

Ii

Die Herstellung von Verbindungen der Formel I wird beispielhaft durch das folgende Formelschema erläutert:

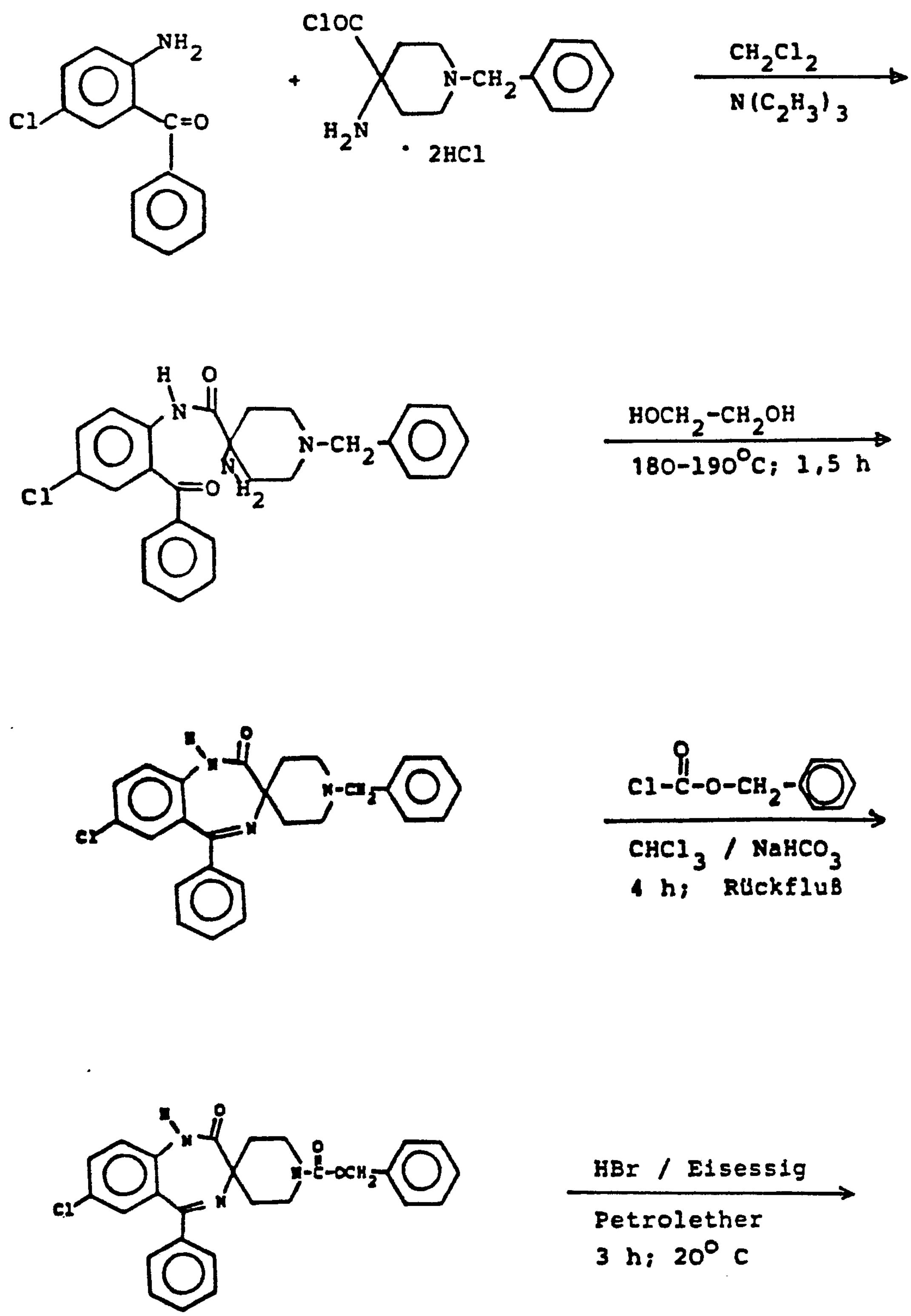

$CH_2Cl_2$
$N(C_2H_3)_3$
$HOCH_2-CH_2OH$
180-190°C; 1,5 h
$CHCl_3$ / $NaHCO_3$
4 h; Rückfluß
HBr / Eisessig
Petrolether
3 h; 20° C

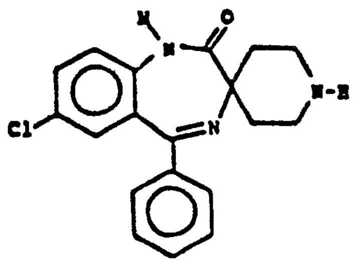

Die folgenden Reaktionsgleichungen sollen die Herstellung weiterer Verbindungen der Formel I beispielhaft veranschaulichen:

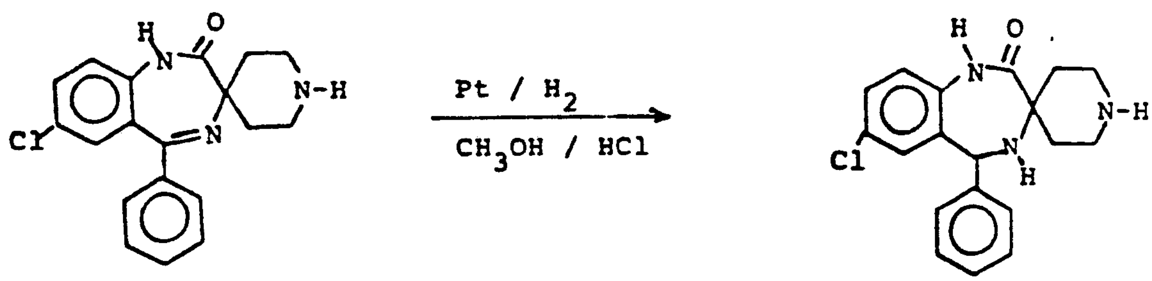


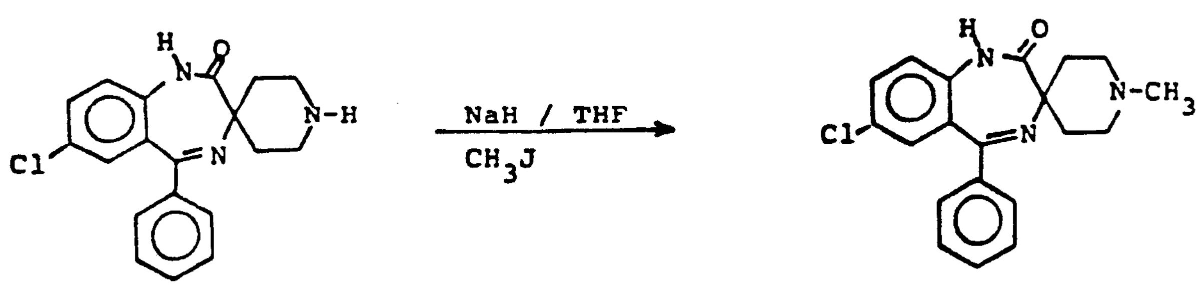


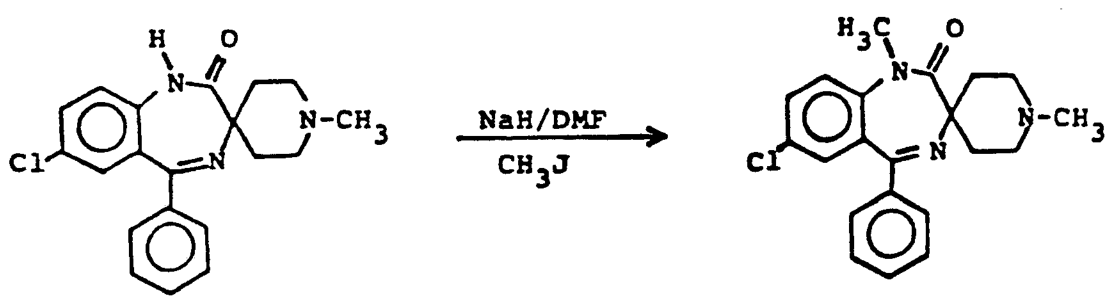

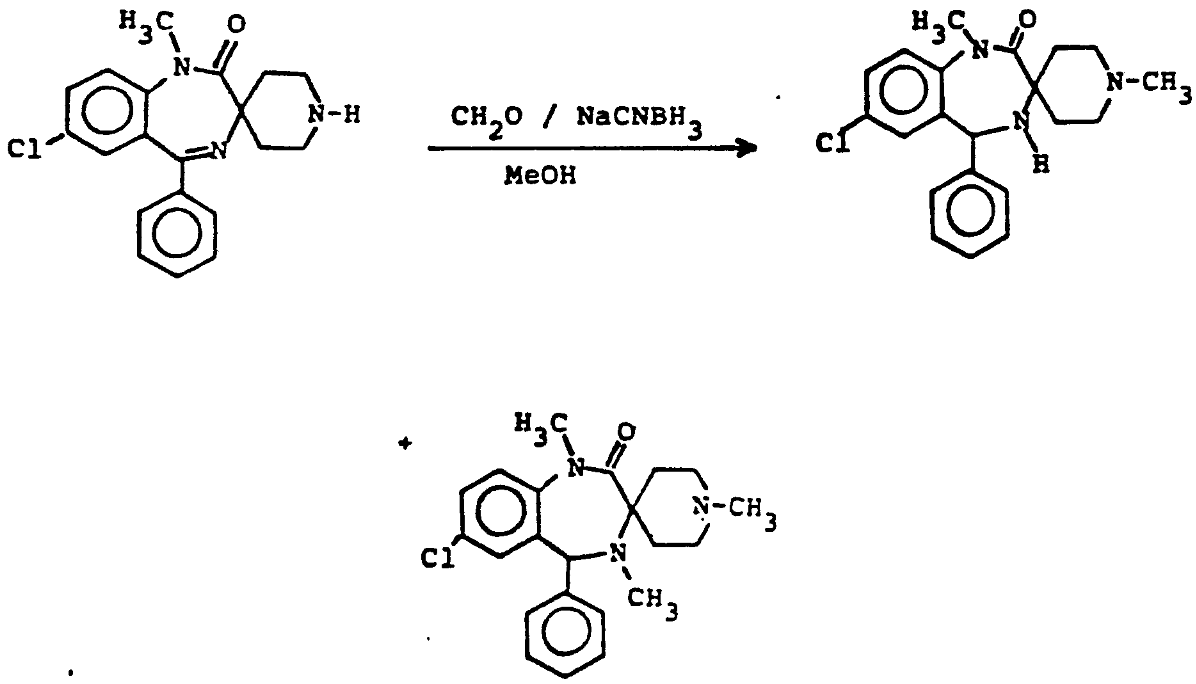
CH2O / NaCNBH3
MeOH

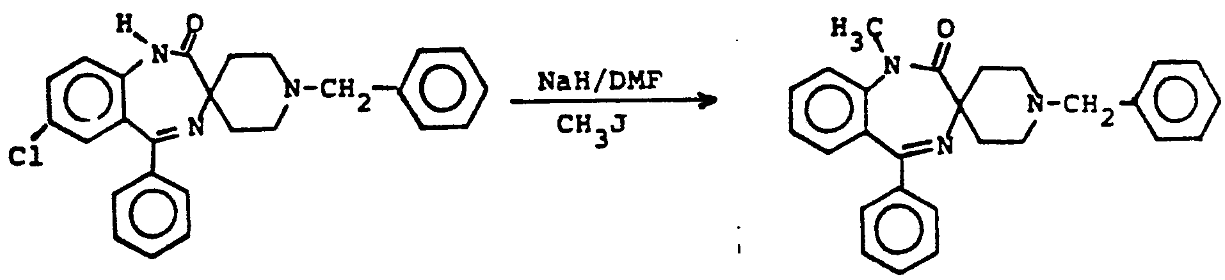
NaH/DMF
CH3J

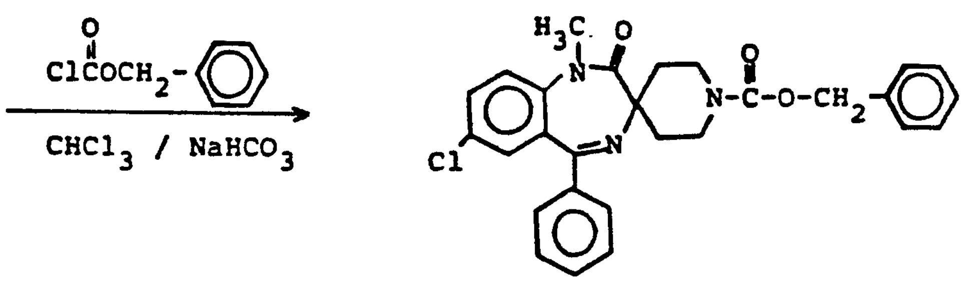
CHCl3 / NaHCO3

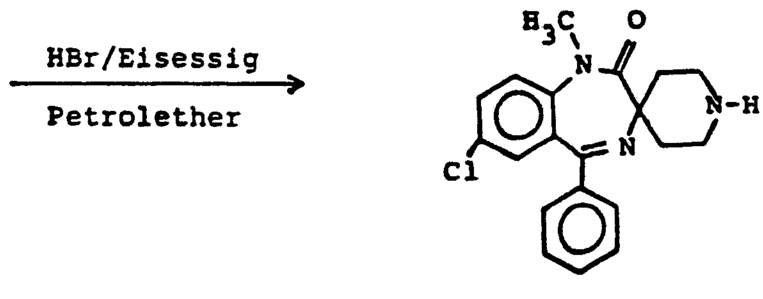


Die Umsetzung der Aminoketone der Formel II (Z = H) mit Säurechloriden der Formel III (Y = H, X = Cl) erfolgt in einem inerten Lösungsmittel in Gegenwart einer Base zum Abfangen der Salzsäure. Bevorzugte Lösungsmittel sind chlorierte Kohlenwasserstoffe, wie Methylenchlorid oder Dichlorethan, Ether, wie Tetrahydrofuran, Dioxan oder Diethylether, oder aromatische Kohlenwasserstoffe, wie Toluol. Als Hilfsbasen kommen tertiäre Amine, wie Triethylamin, oder anorganische Basen, wie $NaHCO_3$ oder Pottasche, in Betracht. Die Säurechloride werden in Form ihrer Bishydrochloride in die Reaktion eingesetzt. Die Umsetzung erfolgt bei Temperaturen zwischen 0°C und 50°C, bevorzugt bei Raumtemperatur.

Der zweite Reaktionsschritt zur Herstellung von Verbindungen der Formel IV aus Verbindungen der Formel II (Z = H) und III (Y = H, X = Cl), die cyclisierende Knüpfung der C = N-Doppelbindung, erfolgt durch Erhitzen der offenkettigen Zwischenprodukte in einem inerten, hochsiedenden Lösungsmittel. Bevorzugte Lösungsmittel sind Dichlorbenzol oder Tetralin, besonders bevorzugt ist die Verwendung von Ethylenglykol. Die Reaktion wird bei Temperaturen zwischen 130°C und 230°C durchgeführt, bevorzugt in einem Temperaturbereich zwischen 150° und 200°C. Das sich bildende Wasser kann gegebenenfalls azeotrop entfernt werden. Gegebenenfalls kann die Wasserabspaltung durch Säuren, wie p-Toluolsulfonsäure, katalysiert werden.

Die Abspaltung der Schutzgruppe in den Verbindungen der Formel IV erfolgt bevorzugt in zwei Schritten, wobei zuerst durch Umsetzung der Verbindungen IV mit Chlorkohlensäurebenzylester in einem inerten organischen Lösungsmittel in Gegenwart einer anorganischen Hilfsbase der Schutzgruppenrest durch einen Carbobenzoxyrest ersetzt wird. Als Lösungsmittel werden bevorzugt chlorierte Kohlenwasserstoffe, wie Chloroform oder Dichlorethan, verwendet, als anorganische Hilfsbasen Alkalicarbonate oder -hydrogencarbonate, wie $K_2CO_3$ oder $NaHCO_3$. Die Reaktion wird bei Temperaturen zwischen Raumtemperatur und der Siedetemperatur des Lösungsmittels, bevorzugt im Temperaturbereich von 40° bis 100°C, durchgeführt. Im zweiten Schritt erfolgt dann die Abspaltung der Carbobenzoxyschutzgruppe, bevorzugt durch eine Umsetzung mit HBr/Eisessig in Gegenwart eines inerten Lösungsmittels.

Als Lösungsmittel wird die Verwendung von Petrolether besonders bevorzugt. Die Reaktion wird in Temperaturbereich von 0° bis 60°C, bevorzugt bei Raumtemperatur durchgeführt. Bevorzugt ist die Verwendung von mit HBr gesättigtem Eisessig zur Abspaltung der Carbobenzoxyschutzgruppe.

Die Absättigung der Doppelbindung des Siebenrings von Verbindungen der Formel I mit Wasserstoff kann durch katalytische Hydrierung erfolgen oder durch eine Umsetzung mit komplexen Metallhydriden, wie $NaBH_4$. Bevorzugt ist die katalytische Hydrierung mit Platin als Katalysator bei Raumtemperatur und Normaldruck. Als Lösungsmittel finden bevorzugt Alkohole Verwendung; besonders bevorzugt sind Alkohole, wie Methanol in Gegenwart von Salzsäure.

Die Alkylierung der drei verschiedenen NH-Funktionen in Verbindungen der Formel I kann durch Umsetzung mit Alkylhalogeniden in Gegenwart einer Hilfsbase, wie $K_2CO_3$ oder $NaHCO_3$, oder nach vorheriger Salzbildung mit NaH erfolgen oder durch eine reduktive Alkylierung mit Aldehyden oder Ketonen in Gegenwart von $NaCNBH_3$. Als Lösungsmittel für die Alkylierung mit Alkylhalogeniden finden bevorzugt aprotische Lösungsmittel, wie Tetrahydrofuran oder Dimethylformamid, Verwendung, für die reduktive Alkylierung werden Alkohole, insbesondere Methanol, bevorzugt. Die Reaktion wird bei Temperaturen zwischen 0°C und der Siedetemperatur des Lösungsmittels durchgeführt; bevorzugt wird die Reaktion bei Raumtemperatur durchgeführt.

Die Aminoketone der Formel II (Z = H) sind bekannt oder können, soweit sie neu sind, in Analogie zu an sich bekannten Methoden, wie sie z. B. in Ehrhart, Ruschig, Arzneimittel, 2. Auflage, Band 1, S. 262, Verlag Chemie, Weinheim/Bergstraße, 1972, beschrieben werden, hergestellt werden.

Die Aminosäurederivate der Formel III sind ebenfalls bekannt oder können ebenfalls in Analogie zu an sich bekannten Verfahren synthetisiert werden (DOS-2 215 721).

Als neue erfindungsgemäße Benzodiazepine seien im einzelnen genannt:

7-Chlor-5-phenyl-spiro[1H-1,4-benzodiazepin-3,4'-piperidin]-2(3H)-on
7-Chlor-5-phenyl-1'-benzyl-spiro[1H-1,4-benzodiazepin-3,4'-piperidin]-2(3H)-on
7-Chlor-5-phenyl-1-methyl-spiro[1H-1,4-benzodiazepin-3,4'-piperidin]-2(3H)-on
7-Chlor-5-phenyl-1'-methyl-spiro[1H-1,4-benzodiazepin-3,4'-piperidin]-2(3H)-on
7-Chlor-5-phenyl-1'-benzyl-1-methyl-spiro[1H-1,4-benzodiazepin-3,4'-piperidin]-2(3H)-on
7-Chlor-5-phenyl-1,1'-dimethyl-spiro[1H-1,4-benzodiazepin-3,4'-piperidin]-2(3H)-on
5-Methyl-1'-benzyl-spiro[1H-1,4-benzodiazepin-3,4'-piperidin]-2(3H)-on

7-Chlor-4,5-dihydro-5-phenyl-spiro[1H-1,4-benzodiazepin-3,4'-piperidin]-2(3H)-on
7-Chlor-4,5-dihydro-5-phenyl-1-ethyl-spiro[1H-1,4-benzodiazepin-3,4'-piperidin]-2(3H)-on
7-Chlor-5-phenyl-1-ethyl-spiro[1H-1,4-benzodiazepin-3,4'-piperidin]-2(3H)-on
7-Chlor-4,5-dihydro-5-phenyl-1'-methyl-spiro[1H-1,4-benzodiazepin-3,4'-piperidin]-2(3H)-on
7-Chlor-4,5-dihydro-5-phenyl-1,1'-dimethyl-spiro[1H-1,4-benzodiazepin-3,4'-piperidin]-2(3H)-on
7-Chlor-4,5-dihydro-5-phenyl-1,4,1'-trimethyl-spiro[1H-1,4-benzodiazepin-3,4'-piperidin]-2(3H)-on
7-Chlor-4,5-dihydro-5-phenyl-4,1'-dimethyl-spiro[1H-1,4-benzodiazepin-3,4'-piperidin]-2(3H)-on
7-Chlor-4,5-dihydro-5-phenyl-1-methyl-spiro[1H-1,4-benzodiazepin-3,4'-piperidin]-2(3H)-on
7-Chlor-5-(4-chlorphenyl)-1'-benzyl-spiro[1H-1,4-benzodiazepin-3,4'-piperidin]-2(3H)-on
5-Phenyl-1'-benzyl-spiro[1H-1,4-benzodiazepin-3,4'-piperidin]-2(3H)-on
5-(2-Fluorphenyl)-1'-benzyl-spiro[1H-1,4-benzodiazepin-3,4'-piperidin]-2(3H)-on
7-Chlor-5-(2-chlorphenyl)-1'-benzyl-spiro[1H-1,4-benzodiazepin-3,4'-piperidin]-2(3H)-on
7-Chlor-5-(2-fluorphenyl)-1'-benzyl-spiro[1H-1,4-benzodiazepin-3,4'-piperidin]-2(3H)-on
8-Methyl-5-phenyl-1'-benzyl-spiro[1H-1,4-benzodiazepin-3,4'-piperidin]-2(3H)-on
7-Chlor-5-(2-thienyl)-1'-benzyl-spiro[1H-1,4-benzodiazepin-3,4'-piperidin]-2(3H)-on
7-Chlor-5-(2-pyridyl)-1'-benzyl-spiro[1H-1,4-benzodiazepin-3,4'-piperidin]-2(3H)-on
7-Chlor-5-cyclohexyl-1'-benzyl-spiro[1H-1,4-benzodiazepin-3,4'-piperidin]-2(3H)-on
7-Chlor-5-isopropyl-1'-benzyl-spiro[1H-1,4-benzodiazepin-3,4'-piperidin]-2(3H)-on
7-Chlor-5-butyl-1'-benzyl-spiro[1H-1,4-benzodiazepin-3,4'-piperidin]-2(3H)-on
5-(4-Methylphenyl)-1'-benzyl-spiro[1H-1,4-benzodiazepin-3,4'-piperidin]-2(3H)-on
7-Nitro-5-phenyl-1'-benzyl-spiro[1H-1,4-benzodiazepin-3,4'-piperidin]-2(3H)-on
5-Methyl-spiro[1H-1,4-benzodiazepin-3,4'-piperidin]-2(3H)-on
Spiro[1H-1,4-benzodiazepin-3,4'-piperidin]-2(3H)-on
7-Chlor-5-(4-chlorphenyl)-spiro[1H-1,4-benzodiazepin-3,4'-piperidin]-2(3H)-on
5-Phenyl-spiro[1H-1,4-benzodiazepin-3,4'-piperidin]-2(3H)-on
5-(2-Fluorphenyl)-spiro[1H-1,4-benzodiazepin-3,4'-piperidin]-2(3H)-on
7-Chlor-5-(2-fluorphenyl)-spiro[1H-1,4-benzodiazepin-3,4'-piperidin]-2(3H)-on
7-Chlor-5-(2-chlorphenyl)-spiro[1H-1,4-benzodiazepin-3,4'-piperidin]-2(3H)-on
8-Methyl-5-phenyl-spiro[1H-1,4-benzodiazepin-3,4'-piperidin]-2(3H)-on
7-Chlor-5-(2-thienyl)-spiro[1H-1,4-benzodiazepin-3,4'-piperidin]-2(3H)-on
7-Chlor-5-(2-pyridyl)-spiro[1H-1,4-benzodiazepin-3,4'-piperidin]-2(3H)-on
7-Chlor-5-cyclohexyl-spiro[1H-1,4-benzodiazepin-3,4'-piperidin]-2(3H)-on
7-Chlor-5-isopropyl-spiro[1H-1,4-benzodiazepin-3,4'-piperidin]-2(3H)-on
7-Chlor-5-butyl-spiro[1H-1,4-benzodiazepin-3,4'-piperidin]-2(3H)-on
5-(4-Methylphenyl)-spiro[1H-1,4-benzodiazepin-3,4'-piperidin]-2(3H)-on
7-Nitro-5-phenyl-spiro[1H-1,4-benzodiazepin-3,4'-piperidin]-2(3H)-on
7-Chlor-5-(3-furyl)-spiro[1H-1,4-benzodiazepin-3,4'-piperidin]-2(3H)-on
7-Chlor-1-methyl-5-(3-furyl)-spiro[1H-1,4-benzodiazepin-3,4'-piperidin]-2(3H)-on
7-Chlor-5-(2-thiazolyl)-spiro[1H-1,4-benzodiazepin-3,4'-piperidin]-2(3H)-on
7-Chlor-5-(1-methyl-2-pyrrolyl)-spiro[1H-1,4-benzodiazepin-3,4'-piperidin]-2(3H)-on
7-Chlor-5-(2-furyl)-spiro[1H-1,4-benzodiazepin-3,4'-piperidin]-2(3H)-on
7-Chlor-1-methyl-5-(2-furyl)-spiro[1H-1,4-benzodiazepin-3,4'-piperidin]-2(3H)-on
7-Chlor-4,5-dihydro-5-(2-furyl)-spiro[1H-1,4-benzodiazepin-3,4'-piperidin]-2(3H)-on
7-Chlor-5-(3-thienyl)-spiro[1H-1,4-benzodiazepin-3,4'-piperidin]-2(3H)-on
7-Chlor-5-(3,5-dimethyl-4-isoxazolyl)-spiro[1H-1,4-benzodiazepin-3,4'-piperidin]-2(3H)-on
7-Chlor-1-methyl-5-(3,5-dimethyl-4-isoxazolyl)-spiro[1H-1,4-benzodiazepin-3,4'-piperidin]-2(3H)-on
7-Chlor-5-(1-methyl-5-pyrazolyl)-spiro[1H-1,4-benzodiazepin-3,4'-piperidin]-2(3H)-on
7-Chlor-1-methyl-5-(1-methyl-5-pyrazolyl)-spiro[1H-1,4-benzodiazepin-3,4'-piperidin]-2(3H)-on
7-Chlor-5-(1-methyl-2-imidazolyl)-spiro[1H-1,4-benzodiazepin-3,4'-piperidin]-2(3H)-on
7-Chlor-5-(3-pyridyl)-spiro[1H-1,4-benzodiazepin-3,4'-piperidin]-2(3H)-on
7-Chlor-5-(4-pyridyl)-spiro[1H-1,4-benzodiazepin-3,4'-piperidin]-2(3H)-on
7-Chlor-1-ethyl-5-(4-pyridyl)-spiro[1H-1,4-benzodiazepin-3,4'-piperidin]-2(3H)-on
7-Chlor-5-(cyclohexen-1-yl)-spiro[1H-1,4-benzodiazepin-3,4'-piperidin]-2(3H)-on
7-Chlor-spiro[1H-1,4-benzodiazepin-3,4'-piperidin]-2(3H)-on
7-Chlor-1-methyl-spiro[1H-1,4-benzodiazepin-3,4'-piperidin]-2(3H)-on
7-Chlor-1-methyl-4,5-dihydro-spiro[1H-1,4-benzodiazepin-3,4'-piperidin]-2(3H)-on
7-Chlor-5-methyl-spiro[1H-1,4-benzodiazepin-3,4'-piperidin]-2(3H)-on
7-Chlor-4,5-dihydro-5-methyl-spiro[1H-1,4-benzodiazepin-3,4'-piperidin]-2(3H)-on
7-Chlor-1,5-dimethyl-spiro[1H-1,4-benzodiazepin-3,4'-piperidin]-2(3H)-on
7-Chlor-4,5-dihydro-1,5-dimethyl-spiro[1H-1,4-benzodiazepin-3,4'-piperidin]-2(3H)-on
7-Chlor-5-ethyl-spiro[1H-1,4-benzodiazepin-3,4'-piperidin]-2 (3H)-on
7-Chlor-5-cyclopropyl-spiro[1H-1,4-benzodiazepin-3,4'-piperidin]-2 (3H) -on
7-Chlor-5-cyclopropylmethyl-spiro[1H-1,4-benzodiazepin-3,4'-piperidin]-2(3H)-on
7-Chlor-5-benzyl-spiro[1H-1,4-benzodiazepin-3,4'-piperidin]-2(3H)-on
5-Phenethyl-spiro[1H-1,4-benzodiazepin-3,4'-piperidin]-2(3H)-on

7-Chlor-5-phenethyl-spiro[1H-1,4-benzodiazepin-3,4'-piperidin]-2(3H)-on
7-Chlor-5-cinnamyl-spiro[1H-1,4-benzodiazepin-3,4'-piperidin]-2(3H)-on
7-Chlor-5-allyl-spiro[1H-1,4-benzodiazepin-3,4'-piperidin]-2(3H)-on
7-Chlor-5-(2-pyridylmethyl)-spiro[1H-1,4-benzodiazepin-3,4'-piperidin]-2(3H)-on
7-Chlor-5-(4-pyridylmethyl)-spiro[1H-1,4-benzodiazepin-3,4'-piperidin]-2(3H)-on
7-Chlor-5-(2-chlor-3-thienyl)-spiro[1H-1,4-benzodiazepin-3,4'-piperidin]-2(3H)-on
7-Chlor-5-(5-isothiazolyl)-spiro[1H-1,4-benzodiazepin-3,4'-piperidin]-2(3H)-on
7-Chlor-5-(4-cyanphenyl)-spiro[1H-1,4-benzodiazepin-3,4'-piperidin]-2(3H)-on
7-Chlor-5-(2,4-dichlorphenyl)-spiro[1H-1,4-benzodiazepin-3,4'-piperidin]-2(3H)-on
5-(2,4-Dichlorphenyl)-spiro[1H-1,4-benzodiazepin-3,4'-piperidin]-2(3H)-on
7-Chlor-4,5-dihydro-5-(2,4-dichlorphenyl)-spiro[1H-1,4-benzodiazepin-3,4'-piperidin]-2(3H)-on
7-Chlor-5-(2-iodphenyl)-spiro[1H-1,4-benzodiazepin-3,4'-piperidin]-2(3H)-on
7-Chlor-5-(3-iodphenyl)-spiro[1H-1,4-benzodiazepin-3,4'-piperidin]-2(3H)-on
7-Chlor-5-(4-iodphenyl)-spiro[1H-1,4-benzodiazepin-3,4'-piperidin]-2(3H)-on
7-Chlor-5-(2-nitrophenyl)-spiro[1H-1,4-benzodiazepin-3,4'-piperidin]-2(3H)-on
7-Chlor-5-(3-nitrophenyl)-spiro[1H-1,4-benzodiazepin-3,4'-piperidin]-2(3H)-on
7-Chlor-5-(4-nitrophenyl)-spiro[1H-1,4-benzodiazepin-3,4'-piperidin]-2(3H)-on
7-Chlor-5-(4-methylphenyl)-spiro[1H-1,4-benzodiazepin-3,4'-piperidin]-2(3H)-on
7-Chlor-4,5-dihydro-5-(4-methylphenyl)-spiro[1H-1,4-benzodiazepin-3,4'-piperidin]-2(3H)-on
1-Methyl-5-(4-methylphenyl)-spiro[1H-1,4-benzodiazepin-3,4'-piperidin]-2(3H)-on
7-Nitro-1-methyl-5-phenyl-spiro[1H-1,4-benzodiazepin-3,4'-piperidin]-2(3H)-on
7-Nitro-1-ethyl-5-phenyl-spiro[1H-1,4-benzodiazepin-3,4'-piperidin]-2(3H)-on
7-Trifluormethyl-5-phenyl-spiro[1H-1,4-benzodiazepin-3,4'-piperidin]-2(3H)-on
7-Trifluormethyl-4,5-dihydro-5-phenyl-spiro[1H-1,4-benzodiazepin-3,4'-piperidin]-2(3H)-on
7-Methoxy-5-phenyl-spiro[1H-1,4-benzodiazepin-3,4'-piperidin]-2(3H)-on
7-Methoxy-4,5-dihydro-5-phenyl-spiro[1H-1,4-benzodiazepin-3,4'-piperidin]-2(3H)-on
7,9-Dichlor-5-phenyl-spiro[1H-1,4-benzodiazepin-3,4'-piperidin]-2(3H)-on
7,9-Dichlor-1-methyl-5-phenyl-spiro[1H-1,4-benzodiazepin-3,4'-piperidin]-2(3H)-on
7-Cyan-5-phenyl-spiro[1H-1,4-benzodiazepin-3,4'-piperidin]-2(3H)-on
7-Cyan-1-methyl-5-phenyl-spiro[1H-1,4-benzodiazepin-3,4'-piperidin]-2(3H)-on
7-Brom-5-phenyl-spiro[1H-1,4-benzodiazepin-3,4'-piperidin]-2(3H)-on
8-Nitro-5-phenyl-spiro[1H-1,4-benzodiazepin-3,4'-piperidin]-2(3H)-on
9-Nitro-5-phenyl-spiro[1H-1,4-benzodiazepin-3,4'-piperidin]-2(3H)-on
6,9-Dichlor-5-phenyl-spiro[1H-1,4-benzodiazepin-3,4'-piperidin]-2(3H)-on
8-Cyan-5-phenyl-spiro[1H-1,4-benzodiazepin-3,4'-piperidin]-2(3H)-on
8-Ethyl-5-phenyl-spiro[1H-1,4-benzodiazepin-3,4'-piperidin]-2(3H)-on
6,8-Dimethyl-5-phenyl-spiro[1H-1,4-benzodiazepin-3,4'-piperidin]-2(3H)-on
7,8-Dimethoxy-5-phenyl-spiro[1H-1,4-benzodiazepin-3,4'-piperidin]-2(3H)-on
8-Trifluormethyl-5-phenyl-spiro[1H-1,4-benzodiazepin-3,4'-piperidin]-2(3H)-on
7-Chlor-5-(2-methoxyphenyl)-spiro[1H-1,4-benzodiazepin-3,4'-piperidin]-2(3H)-on
7-Chlor-5-(3-methoxyphenyl)-spiro[1H-1,4-benzodiazepin-3,4'-piperidin]-2(3H)-on
7-Chlor-5-(4-methoxyphenyl)-spiro[1H-1,4-benzodiazepin-3,4'-piperidin]-2(3H)-on
7-Trifluorphenyl-5-(2-methoxyphenyl)-spiro[1H-1,4-benzodiazepin-3,4'-piperidin]-2(3H)-on
8-Cyan-5-(3-methoxyphenyl)-spiro[1H-1,4-benzodiazepin-3,4'-piperidin]-2(3H)-on
7-Nitro-5-(4-methoxyphenyl)-spiro[1H-1,4-benzodiazepin-3,4'-piperidin]-2(3H)-on
7-Chlor-5-(2-trifluormethylphenyl)-spiro[1H-1,4-benzodiazepin-3,4'-piperidin]-2(3H)-on
7-Chlor-5-(3-trifuormethylphenyl)-spiro[1H-1,4-benzodiazepin-3,4'-piperidin]-2(3H)-on
7-Chlor-5-(4-trifluormethylphenyl)-spiro[1H-1,4-benzodiazepin-3,4'-piperidin]-2(3H)-on
7-Trifluormethyl-5-(2-trifluormethylphenyl)-spiro[1H-1,4-benzodiazepin-3,4'-piperidin]-2(3H)-on
5-(2-Methoxyphenyl)-spiro[1H-1,4-benzodiazepin-3,4'-piperidin]-2(3H)-on
9-Cyan-7,8-dimethyl-5-phenyl-spiro[1H-1,4-benzodiazepin-3,4'-piperidin]-2(3H)-on
7-Chlor-5-(4-ethylphenyl)-spiro[1H-1,4-benzodiazepin-3,4'-piperidin]-2(3H)-on
7-Chlor-5-(2-furyl)-1'-benzyl-spiro[1H-1,4-benzodiazepin-3,4'-piperidin]-2(3H)-on
7-Chlor-5-(2-thiazolyl)-1'-benzyl-spiro[1H-1,4-benzodiazepin-3,4'-piperidin]-2(3H)-on
7-Chlor-5-methyl-1'-benzyl-spiro[1H-1,4-benzodiazepin-3,4'-piperidin]-2(3H)-on
7-Brom-5-phenyl-1'-benzyl-spiro[1H-1,4-benzodiazepin-3,4'-piperidin]-2(3H)-on
7-Trifluormethyl-5-phenyl-1'-benzyl-spiro[1H-1,4-benzodiazepin-3,4'-piperidin]-2(3H)-on
7-Chlor-5-(3-iodphenyl)-1'-benzyl-spiro[1H-1,4-benzodiazepin-3,4'-piperidin]-2(3H)-on
7-Chlor-5-phenyl-spiro[1H-1,4-benzodiazepin-3,3'-pyrrolidin]-2(3H)-on
1',2',3',5',6',7'-Hexahydro-7-chlor-5-phenyl-spiro[1H-1,4-benzodiazepin-3,4'-azepin]-2(3H)-on

Die lern- und gedächtnisverbessernde Wirkung von erfindungsgemäßen Wirksubstanzen wurde z. B. in folgenden Tests untersucht:

### 1. Lernen eines aktiven Vermeidungsverhaltens

Ratten werden in einer "two-way-avoidance Box" ("shuttle box") darauf trainiert, nach Präsentation eines Tones und eines Lichtsignals (CS) innerhalb von 10 Sekunden auf die andere Seite des Testkäfigs zu laufen (CR). Falsche Reaktion oder das Ausbleiben einer solchen wird mit einem Fußschock (UCS; 0,5 mA, max. 10 Sek.) bestraft (Literatur: Y. Tamaki and Y. Kameyama, Pharmac. Biochem. Behav. 16, 1982, 943-947). An jedem Versuchstag finden 20 Durchgänge (Trials) statt. Die Intervall-Zeit zwischen den Trials beträgt 20 - 60 Sekunden. Insgesamt wird an 3 aufeinanderfolgenden Tagen trainiert. Prüfsubstanzen, die nach täglicher Gabe 15 oder 30 Minuten vor Testbeginn die Anzahl der Vermeidungsreaktionen im Verlaufe des Versuches erhöhen oder die Vermeidungslatenzen (Zeit zwischen CS und CR) im Vergleich zu Kontrollen verkürzen, wird eine lernverbessernde Wirkung zugeschrieben.

Ein Beispiel ist in Tabelle 1 dargestellt:

**Tabelle 1**

| Versuchs-tag | Mittelwert der Vermeidungslatenzen in Sekunden je 20 Trials, n = 10) | |
|---|---|---|
| | Kontrolle | Beispiel 8 (10 kg/kg/d, p.o.) |
| 1 | 8.2 | 7.1 |
| 2 | 8.0 | 5.4 ** |
| 3 | 6.8 | 4.8 * |

** $p \leqslant 0.01$, $t(28) = 2.82$; * $p \leqslant 0.05$, $t(28) = 2.28$

### 2. Wasserlabyrinth

Männliche Ratten werden in einem Wasserbecken (120 x 50 cm) darauf trainiert, eine Reihe von vertikalen Barrieren zu umschwimmen und den Ausgang des Wasserlabyrinths zu finden (Literatur: C. Giurgea und Mouravieff-Lesuisse, J. Pharmacol. Paris 3, 1972, 17-30). Während der einzelnen Versuchsdurchgänge, die in einem Abstand von 1 bis 3 Tagen durchgeführt werden, wird die Anzahl falscher Schwimmrichtungen gemessen. Die Reduktion der Fehleranzahl bei aufeinanderfolgenden Durchgängen ist Parameter für die Lerngeschwindigkeit der Versuchstiere. Wenn Ratten, die täglich mit einer Prüfsubstanz behandelt werden, eine stärkere Reduktion der Fehlerzahl während des Versuchsablaufs zeigen als unbehandelte Kontrolltiere, wird der Substanz eine lernverbessernde Wirkung zugeschrieben. Solche Versuche werden mit intakten Ratten verschiedenen Alters sowie mit Ratten durchgeführt, bei denen experimentell ein Lerndefizit induziert wurde (z. B. durch elektrische oder chemische Läsionen im Gehirn).

Ein Beispiel ist in Tabelle 2 dargestellt.

**Tabelle 2**

| Versuchs-durchgang | Anzahl der Applika-tionen (je 5 mg/kg i.p.) | Art der Versuchs-tiere, Alter | Mittelwert der Fehleranzahl (n = 10/Gruppe) Kontrolle | Beispiel 1 |
|---|---|---|---|---|
| 1 | 0 | intakt, | 23 | 23 |
| 5 | 4 | 6 Monate | 7 | 3 |
| 1 | 0 | intakt, | 15 | 15 |
| 4 | 5 | 20 Monate | 19 | 8 ** |
| 1 | 0 | Hippocampusläsionen, | 20 | 21 |
| 5 | 4 | 6 Monate | 19 | 12 |
| 1 | 0 | Hippocampusläsionen, | 29 | 29 |
| 7 | 10 | 6 Monate | 19 | 7 |

** p 0.05 Mann-Whitney U-Test 1)
* p 0.01 -"
1) S. Siegel, Nonparametric statistics for the behavioral sciences. Mc Graw-Hill, Kogakusha, Ltd.

Zur vorliegenden Erfindung gehören pharmazeutische Zubereitungen, die neben nicht-toxischen, inerten, pharmazeutisch geeigneten Trägerstoffen eine oder mehrere der erfindungsgemäßen Verbindungen oder deren Salze enthalten oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies

bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie

(a) Füll- und Streckmittel, z. B. Stärken, Milchzucker, Rohrzucker, Glukose, Mannit und Kieselsäure,
(b) Bindemittel, z. B. Carboxymethylcellulose, Alginate, Gelatine, Polyvinylpyrrolidon,
(c) Feuchthaltemittel, z. B. Glycerin,
(d) Sprengmittel, z. B. Agar-Agar, Calciumcarbonat und Natriumcarbonat,
(e) Lösungsverzögerer, z. B. Paraffin und
(f) Resorptionsbeschleuniger, z. B. quarternäre Ammoniumverbindungen,
(g) Netzmittel, z. B. Cetylalkohol, Glycerinmonostearat,
(h) Adsorptionsmittel, z. B. Kaolin und Bentonit und
(i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opalisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett, und höhere Ester (z. B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Ethylalkohol, Propylenglykol, Suspendiermittel, z. B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der Formel (I) und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere oral und intravenös, appliziert werden.

Im allgemeinen hat es sich als vorteilhaft erwiesen, den oder die Wirkstoffe bei parenteraler (i.v. oder i.m.) Applikation in Mengen von etwa 0,01 bis etwa 10, vorzugsweise von 0,1 bis 1 mg/kg Körpergewicht je 24 Stunden und bei oraler Applikation in Mengen von etwa 0,05 bis etwa 100, vorzugsweise 0,1 bis 10 mg/kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von

etwa 0,01 bis etwa 30, insbesondere 0,05 bis 3 mg/kg Körpergewicht.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o. g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

**Beispiel 1**

**7-Chlor-5-phenyl-spiro[1H-1,4-benzodiazepin-3,4'-piperidin]-2(3H)-on**

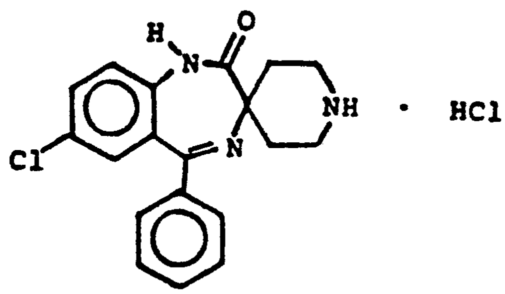

3,5 g (0,0073 Mol) 7-Chlor-5-phenyl-N'-benzyloxycarbonyl-spiro[1H-1,4-benzodiazepin-3,4'-piperidin]-2(3H)-on werden in 50 ml Petrolether (40 - 60°C) suspendiert und bei 0°C unter Rühren mit 6 ml 38 %-iger HBr-Eisessig-Lösung versetzt. Man rührt die Reaktionsmischung 3 Std. bei 20°C, saugt anschließend das entstandene Hydrobromid ab und wäscht 3mal mit Petrolether. Das Salz wird in Wasser gelöst, die wäßrige Lösung mit Diethylether geschüttelt und mit 10 %-iger wäßriger Ammoniaklösung alkalisiert. Die freigesetzte Base wird in Methylenchlorid aufgenommen, die organische Phase über $Na_2SO_4$ getrocknet und über Aktivkohle filtriert. Aus der Methylenchloridlösung wird das Hydrochlorid durch Zugabe einer Diethylether-HCl-Lösung gefällt. Man filtriert, wäscht das Salz mit Diethylether und trocknet bei 155°C im Vakuum über KOH.

Sandfarbenes Pulver vom Fp. 225 - 230°C.

Ausbeute: 73 % der Theorie.

**Herstellung der Ausgangsverbindung:**

**7-Chlor-5-phenyl-1'-benzyloxycarbonyl-spiro[1H-1,4-benzodiazepin-3,4'-piperidin]-2(3H)-on**

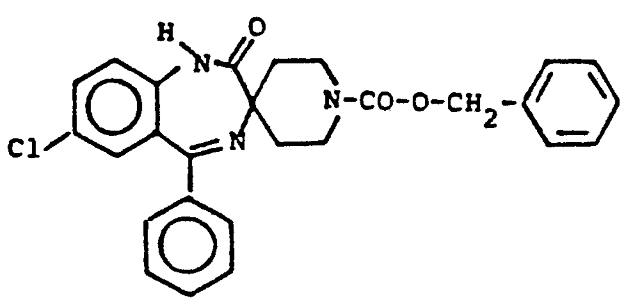

Zu einem Gemisch aus 4,0 g (0,0093 Mol) 7-Chlor-5-phenyl-1'-benzyl-spiro[1H-1,4-benzodiazepin-3,4'-piperidin]-2-(3H)-on und 2,4 g (0,029 Mol) fein gepulvertem $NaHCO_3$ in 80 ml absolutem Chloroform werden 10 g (0,029 Mol) einer 50 %-igen Lösung von Chlorameisensäurebenzylester in Toluol gegeben. Das Reaktionsgemisch wird 3 Std. unter Rückflußkühlung gekocht. Anschließend gießt man auf Eis, trennt die organischen Phase ab und wäscht sie zweimal mit 10 %-iger $NaHCO_3$-Lösung. Nach dem Trocknen über $Na_2SO_4$ wird filtriert und das Lösungsmittel abgedampft. Zurück bleibt ein schwach braun gefärbtes Öl, das mit Petrolether kristallisiert. Die Verbindung wird abgesaugt, mit Petrolether gewaschen und bei 60°C im Vakuum

getrocknet.
Ausbeute: 4,0 g, entsprechend 90 % d. Th.; Fp. 202 - 204° C.

**Beispiel 2**

**7-Chlor-5-phenyl-1'-benzyl-spiro[1H-1,4-benzodiazepin-3,4'-piperidin]-2(3H)-on**

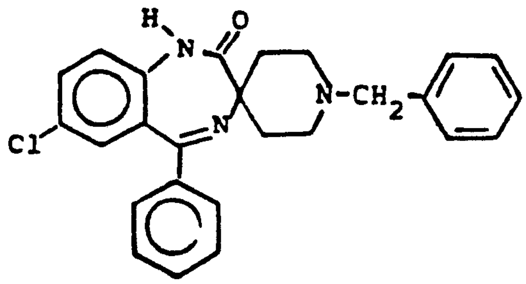

2,0 g (0,0044 Mol) N-(2-Benzoyl-4-chlorphenyl)-4-amino-1 benzylpiperidincarboxamid werden unter Rühren in 10 ml Ethylenglykol 2 Std. auf 180 - 190° C erhitzt. Nach dem Abkühlen auf Raumtemperatur wird mit 30 ml Methylenchlorid verdünnt und die Mischung mit Eiswasser versetzt. Man extrahiert das Reaktionsprodukt zweimal mit Methylenchlorid, trocknet die vereinigten organischen Phasen über $Na_2SO_4$, filtriert und dampft zur Trockene ein. Der Rückstand wird durch Säulenchromatographie an Kieselgel 60 (Merck) gereinigt. Eluiert wird mit einem Gemisch aus Methylenchlorid und Methanol (9 : 1).
Ausbeute: 1,5 g, entsprechend 79 % d. Th.; Fp. 234° C.

**Herstellung der Ausgangsverbindungen:**

a) <u>1-Benzyl-4-amino-4-piperidinocarbonsäure-2'-benzoyl-4'-chloranilid</u>

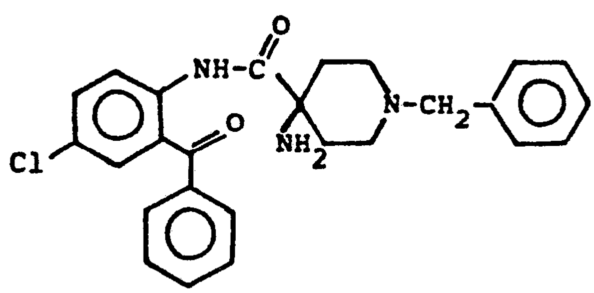

In eine Mischung von 40 g (0,17 Mol) 2-Amino-5-chloracetophenon und 80 g (0,24 Mol) 1-Benzyl-4-amino-4-piperidincarbonsäurechlorid-dihydrochlorid in 900 ml $CH_2Cl_2$ werden bei 0° C unter $N_2$ 60 g (0,6 Mol) Triethylamin unter Rühren zugetropft. Das Gemisch wird eine Stunde bei 0° C und 2 Std. bei 20° C gerührt. Dann wird auf Eis gegossen, mit NaOH auf pH = 10 gestellt und mit $CH_2Cl_2$ extrahiert. Die getrockneten $CH_2Cl_2$-Extrakte werden zur Trockne eingeengt. Zur Reinigung wird der Rückstand an Kieselgel 60 (Merck) chromatografiert. Zuerst wird mit $CH_2Cl_2$/EE 3 : 2 dann mit $CH_2Cl_2$/MeOH 9 : 1 eluiert.
Ausbeute: 46 g entsprechend 51 % d. Th.

b) <u>1-Benzyl-4-amino-4-piperidincarbonsäurechlorid-dihydrochlorid</u>

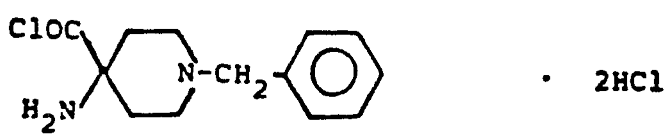

In eine Suspension von 40 g feingepulverter 1-Benzyl-4-amino-4-piperidincarbonsäure in 111 ml $CH_2Cl_2$ wird bei 0° C 1 Std. HCl eingeleitet. Zu dieser Mischung werden unter kräftigem Rühren und weiterem HCl-Einleiten 26 g $PCl_5$ in 200 ml $CH_2Cl_2$ in mehreren Portionen gegeben. Nach 4-stündigem Rühren bei 0° C wird das Reaktionsprodukt über eine Glasfritte unter Feuchtigkeitsausschluß abgesaugt und gründlich mit Petrolether (60 - 80° C) und $CH_2Cl_2$ gewaschen. Das so erhaltene Rohprodukt wird ohne weitere Reinigung in die nächste Reaktionsstufe eingesetzt.

**Beispiel 3**

**7-Chlor-5-phenyl-1-methyl-spiro[1H-1,4-benzodiazepin-3,4'-piperidin]-2(3H)-on**

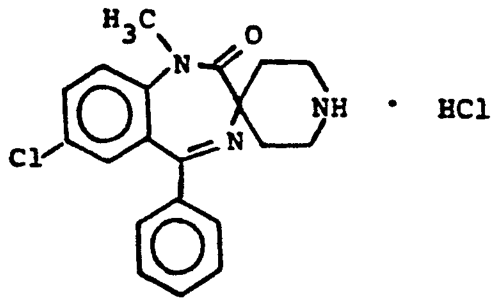

Zu 26 g (0,053 Mol) 7-Chlor-1-methyl-5-phenyl-1'-benzyloxycarbonyl-spiro[1H-1,4-benzodiazepin-3,4'-piperidin]-2(3H)-on in 250 ml Petrolether (40 - 60°C) gibt man unter Rühren bei 0°C 30 ml einer HBr-Eisessig-Lösung (ca. 38 %-ig) hinzu und läßt 8 Std. bei Raumtemperatur reagieren.

Anschließend wird vom überstehenden Petrolether abdekantiert, der viskose Rückstand mit Wasser versetzt und die wäßrige Phase mit wäßriger Ammoniaklösung auf pH 10 eingestellt. Das Reaktionsprodukt wird mit Methylenchlorid extrahiert und durch präparative Säulenchromatografie an Kieselgel 60 (Merck) gereinigt; Elutionsmittel: Methanol-Methylenchlorid (1 : 9) mit Ammoniakzusatz (5 % einer konzentrierten wäßrigen $NH_3$-Lösung).

Durch Aufnehmen der öligen Base in Methylenchlorid und Zugabe einer HCl-Diethylether-Lösung erfolgt die Umwandlung in das kristalline Hydrochlorid.

Ausbeute: 29 % d. Th.; farblose Kristalle vom Fp. 200 - 205°C.

**Herstellung der Ausgangsverbindung:**

Die Ausgangsverbindung 7-Chlor-1-methyl-5-phenyl-1'-benzyloxycarbonyl-spiro[1H-1,4-benzodiazepin-3,4'-piperidin]-2-(3H)-on wird in Analogie zu Beispiel 1 aus 7-Chlor-5-phenyl-1'-benzyl-1-methyl-spiro[1H-1,4-benzodiazepin-3,4'-piperidin]-2(3H)-on erhalten.

**Beispiel 4**

**7-Chlor-5-phenyl-1'-methyl-spiro[1H-1,4-benzodiazepin-3,4'-piperidin]-2(3H)-on**

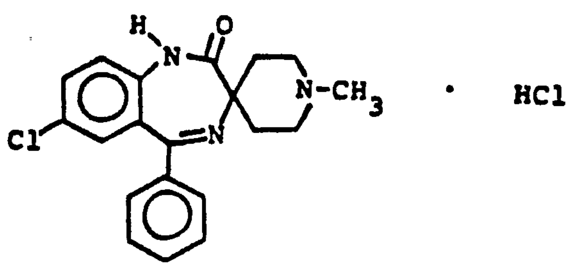

1 g (0,0029 Mol) 7-Chlor-5-phenyl-spiro[1H-1,4-benzodiazepin-3,4'-piperidin]-2(3H)-on und 0,0029 Mol Natriumhydrid (55 - 60 %-ige Dispersion) werden in 30 ml absolutem Tetrahydrofuran 30 Minuten bei 25 C gerührt. Nach Zusatz von 0,41 g (0,0029 Mol) Methyljodid bei 0°C wird 1 Std. bei 0°C, dann eine weitere Stunde bei 25°C gerührt. Anschließend filtriert man die Reaktionsmischung, dampft das Lösungsmittel ab und versetzt mit 50 ml Wasser. Nach Extraktion mit Methylenchlorid wird das Reaktionsprodukt durch Säulenchromatografie an Aluminiumoxid 90, Aktivitätsstufe II - III (Merck) gereinigt; Elutionsmittel: Methylenchlorid-Cyclohexan-Methanol (3 : 6 : 1).

Nach Abdampfen des Lösungsmittels erhält man die ölige Base, die in Diethylether aufgenommen und durch Zugabe von Diethylether-HCl in das Hydrochlorid umgewandelt wird.

Ausbeute: 56 % d. Th.; farblose Kristalle vom Fp. 287°C.

**Beispiel 5**

**7-Chlor-5-phenyl-1'-benzyl-1-methyl-spiro[1H-1,4-benzodiazepin-3,4'-piperidin]-2(3H)-on**

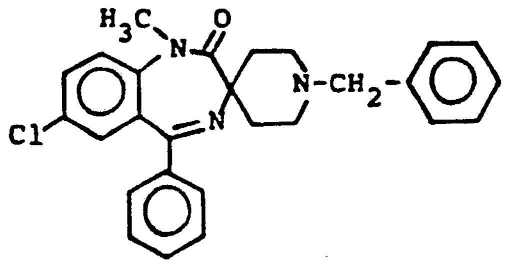

3,0 g (0,0069 Mol) 7-Chlor-5-phenyl-1'-benzyl-spiro[1H-1,4-benzodiazepin-3,4'-piperidin]-2(3H)-on werden in 25 ml absolutem DMF gelöst und bei 25°C mit 0,3 g (0,007 Mol) Natriumhydrid (55 - 60 %-ige Dispersion) versetzt. Nach 20-minütigem Rühren wird auf 0°C abgekühlt, eine Lösung von 1,0 g (0,0065 Mol) Methyljodid in 5 ml absolutem DMF zugetropft und 3 Std. bei 25°C gerührt. Man destilliert das Lösungsmittel ab und reinigt den Rückstand durch Säulenchromatografie über Kieselgel 60 (Merck). Nach dem Eluieren mit Methylenchlorid/Methanol (9 : 1) erhält man die ölige Base, die langsam durchkristallisiert.

Farblose Kristalle vom Fp. 169 - 170°C.

Ausbeute: 2,9 g, entsprechend 96 % d. Th.

**Beispiel 6**

**7-Chlor-5-phenyl-1,1'-dimethyl-spiro[1H-1,4-benzodiazepin-3,4'-piperidin]-2(3H)-on**

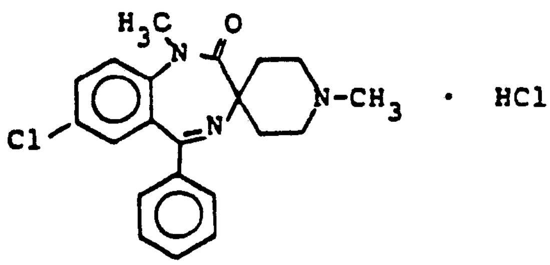

0,6 g (0,0016 Mol) 7-Chlor-5-phenyl-1'-methyl-spiro[1H-1,4-benzodiazepin-3,4'-piperidin]-2(3H)-on werden in 15 ml absolutem DMF gelöst und bei 70°C mit 0,1 g Natriumhydrid (55 - 60 %-ige Dispersion) umgesetzt. Nach 30 Minuten kühlt man auf 25°C ab, versetzt mit 0,22 g (0,0016 Mol) Methyljodid in 10 ml absolutem DMF und rührt 2 Std. Nach dem Abdestillieren des Lösungsmittels nimmt man den Rückstand in Wasser auf und extrahiert mit Methylenchlorid. Zur Reinigung chromatografiert man an Aluminiumoxid 90 (Merck), Aktivitätsstufe II - III, mit einem Cyclohexan-Methylenchlorid-Methanol-Gemisch (6 : 3 : 1) und wandelt die so erhaltene Base durch Behandlung mit Diethylether/HCl in das Hydrochlorid um.

Ausbeute: 0,4 g Hydrochlorid; entsprechend 67 % d. Th.; farbloses Kristallisat mit Fp. 214°C.

**Beispiel 7**

**5-Methyl-1'-benzyl-spiro[1H-1,4-benzodiazepin-3,4'-piperidin]-2(3H)-on**

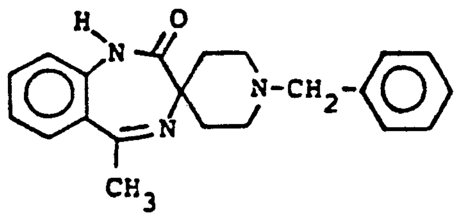

Zu einer Mischung von 5,4 g (0,04 Mol) 2-Aminoacetophenon und 13,0 g (0,04 Mol) 1-Benzyl-4-amino-4-piperidincarbonsäurechlorid-dihydrochlorid (hergestellt aus 9,4 g (0,04 Mol) 1-Benzyl-4-amino-4-carboxypiperidin durch Umsetzung mit überschüssigem Phosphor-V-chlorid in Methylenchlorid bei 25°C) in 400 ml Methylenchlorid tropft man unter Rühren und $N_2$-Atmosphäre bei 0°C die Lösung von 12,2 g (0,12 Mol) Triethylamin in 100 ml Methylenchlorid, rührt 1 Stunde bei 0°C, zuletzt 2 Std. bei 25°C. Dann gießt man auf Eis, stellt mit Natronlauge auf pH 10 und extrahiert das Reaktionsprodukt mit Methylenchlorid. Zur Reinigung chromatografiert man an Kieselgel 60 (Merck), als Elutionsmittel wird ein Methylenchlorid-Methanol-Gemisch (10 : 1) verwendet.

Die eingedampften Eluate liefern ein farbloses Produkt, das aus Methanol umkristallisiert wird.

Ausbeute: 5,0 g, entsprechend 37 % d. Th., Fp. 218 - 219°C.

**Beispiel 8**

**7-Chlor-4,5-dihydro-5-phenyl-spiro[1H-1,4-benzodiazepin-3,4'-piperidin]-2(3H)-on**

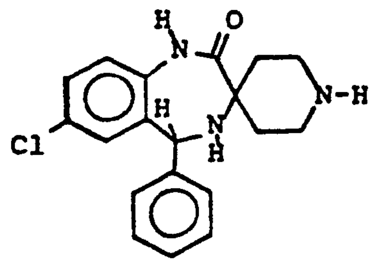

4,0 g (0,012 Mol) 7-Chlor-5-phenyl-spiro[1H-1,4-benzodiazepin-3,4'-piperidin]-2(3H)-on werden in 160 ml Methanol unter Zusatz von 10 ml einer 30 %-igen Isopropanol-HCl-Lösung in Gegenwart von Platin bei 25°C bis zur Aufnahme der berechneten Menge Wasserstoff innerhalb von 3 Std. hydriert. Nach dem Filtrieren wird das Lösungsmittel abdestilliert, der Rückstand mit wäßriger konzentrierter Ammoniaklösung alkalisiert und die Base mit Methylenchlorid ausgeschüttelt. Die mit Wasser gewaschene und über $Na_2SO_4$ getrocknete organische Phase wird eingeengt und an Kieselgel 60 (Merck) chromatografiert;

Elutionsmittel: Methylenchlorid-Methanol-25 %-ige wäßrige Ammoniaklösung (14 : 2,5 : 0,5).

Ausbeute: 3,3 g, entsprechend 80 % d. Th.; Fp. 135°C; farblose Kristalle.

**Beispiel 9**

**7-Chlor-4,5-dihydro-5-phenyl-1-ethyl-spiro[1H-1,4-benzodiazepin-3,4'-piperidin]-2(3H)-on**

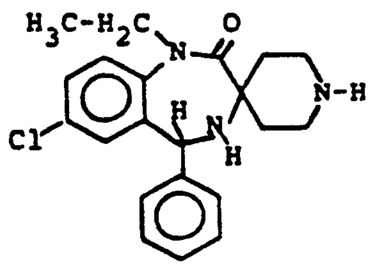

1,5 g (0,0041 Mol) 7-Chlor-5-phenyl-1-ethyl-spiro[1H-1,4-benzodiazepin-3,4'-piperidin]-2(3H)-on werden in 35 ml Methanol unter Zusatz von 4 ml einer 30 %-igen Isopropanol-HCl-Lösung in Gegenwart von Platin bei 25°C bis zur Aufnahme der berechneten Wasserstoffmenge innerhalb von 8 Std. hydriert.

Die nach dem Aufarbeiten erhaltene Base wird mit Diethylether-HCl in das Hydrochlorid überführt.

Ausbeute: 1,0 g, entsprechend 73 % d. Th.; Fp. des Hydrochlorids 212°C.

**Herstellung der Ausgangsverbindung:**

Die Ausgangsverbindung 7-Chlor-5-phenyl-1-ethyl-spiro[1H-1,4-benzodiazepin-3,4'-piperidin]-2(3H)-on wird in Analogie zu Beispiel 3 hergestellt.

**Beispiel 10**

**7-Chlor-4,5-dihydro-5-phenyl-1'-methyl-spiro[1H-1,4-benzodiazepin-3,4'-piperidin]-2(3H)-on**

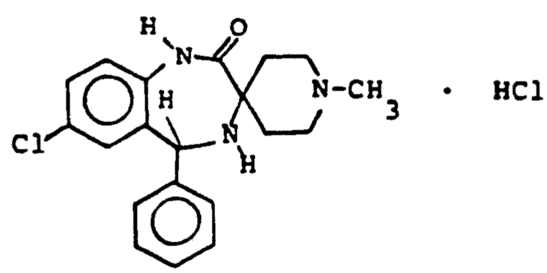

1,2 g (0,003 Mol) 7-Chlor-5-phenyl-1'-methyl-spiro[1H-1,4-benzodiazepin-3,4'-piperidin]-2(3H)-on-hydrochlorid werden in 40 ml Methanol unter Zusatz von 4 ml einer 30 %-igen Isopropanol-HCl-Lösung in Gegenwart von Platin bei 25°C bis zur Aufnahme der berechneten Wasserstoffmenge innerhalb von 2 Std. hydriert.

Das nach dem Aufarbeiten erhaltene Hydrierungsprodukt wird an Kieselgel 60 (Merck) säulenchromatografisch gereinigt. Elutionsmittel: Methylenchlorid/Methanol/ 25 %-ige wäßrige $NH_3$-Lösung (9 : 1 : 0,2).

Die Umwandlung der Base in das Hydrochlorid erfolgt mit Diethylether-HCl. Farblose Kristalle vom Fp. 260 - 270°C.

Ausbeute: 0,8 g Hydrochlorid, entsprechend 68 % d. Th.

**Beispiel 11**

**7-Chlor-4,5-dihydro-5-phenyl-1,1'-dimethyl-spiro[1H-1,4-benzodiazepin-3,4'-piperidin]-2(3H)-on**

und

**7-Chlor-4,5-dihydro-5-phenyl-1,4,1'-trimethyl-spiro[1H-1,4-benzodiazepin-3,4'-piperidin]-2(3H)-on**

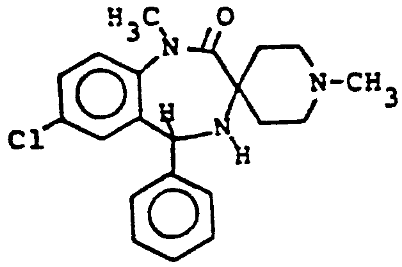 · HCl 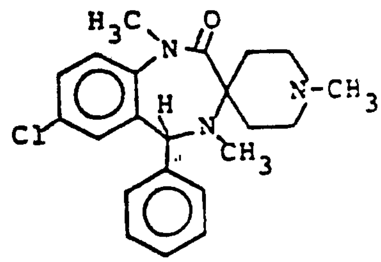

Zu einer Mischung aus 10 g (0,028 Mol) 7-Chlor-5-phenyl-1-methyl-spiro[1H-1,4-benzodiazepin-3,4'-piperidin]-2(3H)-on, 10,8 g (0,12 Mol) Formaldehyd (38 %-ige Lösung in Wasser) und 3,46 g (0,055 Mol) $NaBH_3$ (CN) in 300 ml Methanol werden bei 0°C unter Rühren 3,5 g (0,05 Mol) Eisessig und 30 g Molekularsiebe 3 Å (Merck) gegeben.

Innerhalb von 2 Std. läßt man die Temperatur auf 25°C ansteigen, dampft das Lösungsmittel im Vakuum ab, versetzt den Rückstand mit Eiswasser und stellt durch Zugabe von Salzsäure auf pH 1 ein. Nach beendeter Zersetzung alkalisiert man mit Natronlauge und schüttelt das Reaktionsprodukt mit Essigsäureethylester aus. Zur Reinigung chromatografiert man an Aluminiumoxid 90, Aktivitätsstufe II - III, (Merck) mit einem Lösungsmittelgemisch aus Cyclohexan, Methylenchlorid und Methanol (6 : 3 : 1).

Aus den ersten Fraktionen des Eluats erhält man 0,22 g 7-Chlor-4,5-dihydro-5-phenyl-1,4,1'-trimethyl-spiro[1H-1,4-benzodiazepin-3,4'-piperidin]-2(3H)-on in Form farbloser Kristalle vom Fp. 80 - 85°C.

Ausbeute: 2 % d. Th.

Aus den nachfolgenden Fraktionen werden 6,1 g 7-Chlor-4,5-dihydro-5-phenyl-1,1'-dimethyl-spiro[1H-1,4-benzodiazepin-3,4'-piperidin]-2(3H)-on erhalten. Nach Umwandlung in das Hydrochlorid schmilzt die Substanz bei 196°C.

Ausbeute: 58 % d. Th. (als Hydrochlorid).

**Beispiel 12**

**7-Chlor-4,5-dihydro-5-phenyl-4,1'-dimethyl-spiro[1H-1,4-benzodiazepin-3,4'-piperidin]-2(3H)-on**

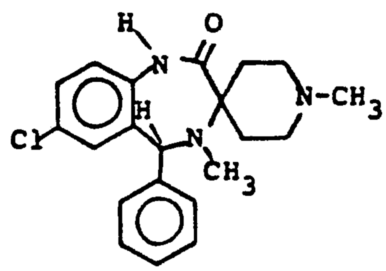 · HCl

1,0 g (0,0029 Mol) 7-Chlor-5-phenyl-spiro[1H-1,4-benzodiazepin-3,4'-piperidin]-2(3H)-on wird in 30 ml Methanol gelöst, die Lösung auf 0°C abgekühlt und nacheinander mit 4,2 g (0,053 Mol) Formaldehyd (38 %-ige Lösung in Wasser), 0,66 g (0,01 Mol) $NaBH_3(CN)$, 1,0 g (0,016 Mol) Eisessig und 3,0 g Molekularsieb 3 Å (Merck) versetzt. Anschließend erhitzt man den Ansatz 7 Std. auf 65°C, dampft das Lösungsmittel im Vakuum ab und säuert durch Zugabe von konz. Salzsäure an. Nach beendeter Zersetzung alkalisiert man mit Natronlauge und extrahiert das Reaktionsprodukt mit Essigsäureethylester. Zur Reinigung chromatografiert man an Kieselgel 60 (Merck) mit einem Lösungsmittelgemisch aus Methylenchlorid, Methanol und konzentrierter wäßriger Ammoniaklösung (9 : 1 : 0,2). Die erhaltene ölige Base wird mit Diethylether-HCl in das Hydrochlorid umgewandelt.

Farblose Kristalle, Fp. 198 - 200°C.

Ausbeute: 35 % d. Th.

**Beispiel 13**

**7-Chlor-4,5-dihydro-5-phenyl-1-methyl-spiro[1H-3,4-benzodiazepin-3,4'-piperidin]-2(3H)-on**

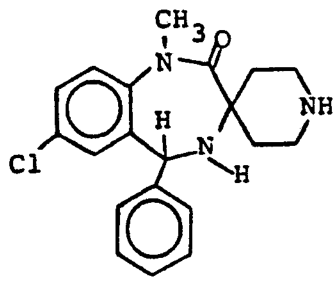

Die Herstellung erfolgte analog zu Beispiel 9 durch Hydrierung von 7-Chlor-5-phenyl-1-methyl-spiro[1H-1,4-benzodiazepin-3,4'-piperidin]-2(3H)-on.

Fp. 188 - 190°C (HCl-Salz).

**Beispiel 14**

**7-Chlor-5-(4-chlorphenyl)-1'-benzyl-spiro[1H-1,4-benzodiazepin-3,4'-piperidin]-2(3H)-on**

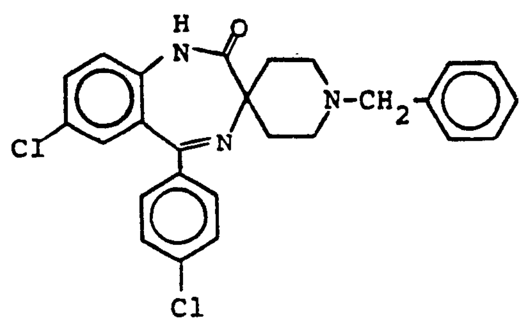

Eine Lösung von 11,4 g (0,043 Mol) 2-Amino-4',5-dichlorbenzophenon in 150 ml Methylenchlorid wird unter Rühren und Eiskühlung zu einer Suspension von 1-Benzyl-4-amino-4-piperidincarbonsäurechloriddihydrochlorid (hergestellt aus 10 g (0,043 Mol) 1-Benzyl-4-amino-4-piperidincarbonsäure, wie oben beschrieben) in 120 ml Methylenchlorid eingetropft.

Anschließend versetzt man tropfenweise mit 18 ml (0,13 Mol) Triethylamin, rührt eine Stunde bei Raumtemperatur und gießt auf Eis. Nach dem Alkalisieren mit 2 n Natronlauge trennt man die organische Phase ab, wäscht mit Wasser und trocknet über $Na_2SO_4$. Die filtrierte Lösung wird eingeengt und der Rückstand säulenchromatographisch an Kieselgel 60 (Merck) gereinigt; Elutionsmittel: Methylenchlorid /Methanol (100 : 3).

Man erhält 10,5g 1-Benzyl-4-amino-4-piperidino-carbonsäure-2'-(4-chlorbenzoyl)-4'-chloranilid, das anschließend durch 8 stündiges Kochen in 200 ml Xylol in Gegenwart von 0,5 g p-Toluolsulfonsäure cyclisiert wird.

Der nach dem Abdestillieren des Lösungsmittels erhaltene Rückstand wird in Methylenchlorid aufgenommen, mit Natronlauge gewaschen und über $Na_2SO_4$ getrocknet. Man erhält ein hellgelbes, amorphes Produkt, das aus Isopropanol kristallisiert.

Ausbeute: 9,5 g, entsprechend 48 % der Theorie (bezogen auf eingesetztes Benzophenon-Derivat); Fp. 140°C.

**Analog zu Beispiel 14 wurden hergestellt:**

| Beisp. Nr. | Formel | Fp. (°C) | (Ausbeute) (% d.Th.) |
|---|---|---|---|
| 15 | 5-Phenyl-1'-benzyl-spiro[1H-1,4-benzodiazepin-3,4'-piperidin]-2(3H)-on<br>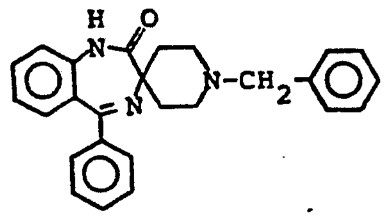 | 209 | 64 |
| 16 | 5-(2-Fluorphenyl)-1'-benzyl-spiro[1H- 1,4-benzodiazepin-3,4'-piperidin]-2(3H)-on<br>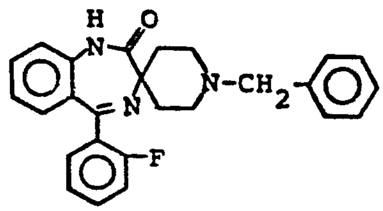 | 215 | 38 |
| 17 | 7-Chlor-5-(2-chlorphenyl)-1'-benzyl-spiro[1H-1,4-benzodiazepin- 3,4'-piperidin]-2(3H)-on<br>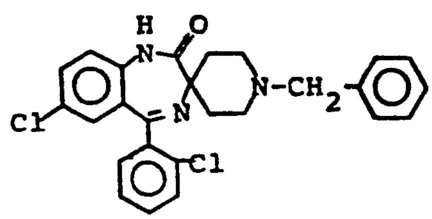 | 246 | 18 |
| 18 | 7-Chlor-5-(2-fluorphenyl)-1'-benzyl-spiro[1H-1,4-benzodiazepin- 3,4'-piperidin]-2(3H)-on<br>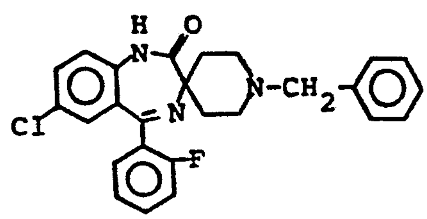 | 206 | 13 |
| 19 | 8-Methyl-5-phenyl-1'-benzyl-spiro[1H-1,4-benzodiazepin- 3,4'-piperidin]-2(3H)-on<br>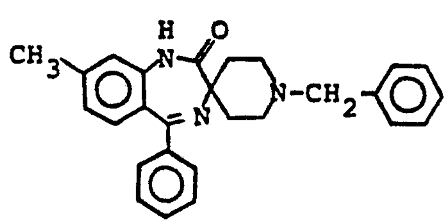 | 273 | 24 |
| 20 | 7-Chlor-5-(2-thienyl)-1'-benzyl-spiro[1H-1,4-benzodiazepin- 3,4'-piperidin]-2(3H)-on | | |

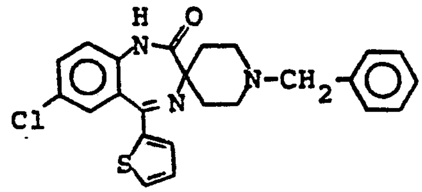 235 81

21 7-Chlor-5-(2-pyridyl)-1'-benzyl-spiro[1H-1,4-benzodiazepin- 3,4'-piperidin]-2(3H)-on

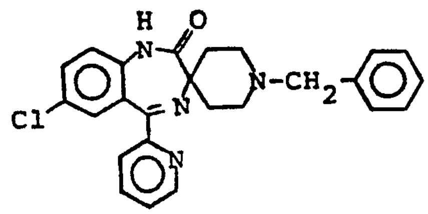 226 73

22 7-Chlor-5-cyclohexyl-1'-benzyl-spiro[1H-1,4-benzodiazepin- 3,4'-piperidin]-2(3H)-on

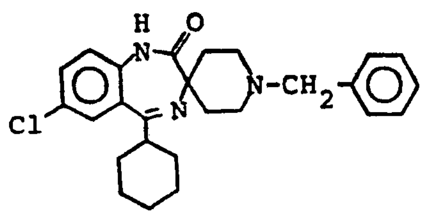 199 23 %

23 7-Chlor-5-isopropyl-1'-benzyl-spiro[1H-1,4-benzodiazepin- 3,4'-piperidin]-2(3H)-on

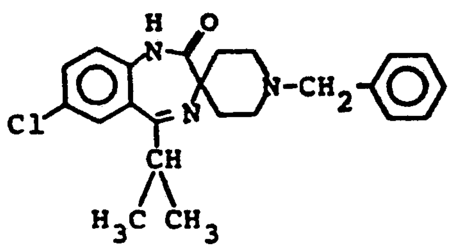 182-183 56

24 7-Chlor-5-butyl-1'-benzyl-spiro[1H-1,4-benzodiazepin- 3,4'-piperidin]-2(3H)-on

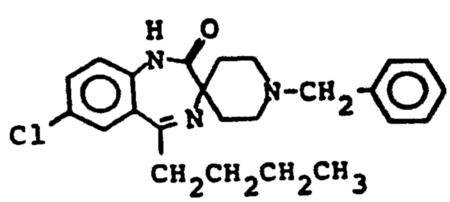 105-106 48

25 5-(4-Methylphenyl)-1'-benzyl-spiro[1H-1,4-benzodiazepin- 3,4'-piperidin]-2(3H)-on

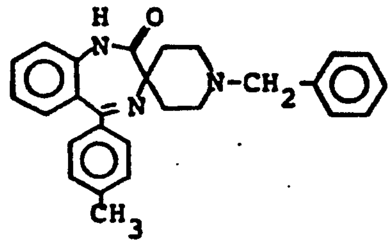 221 18

26 7-Nitro-5-phenyl-1'-benzyl-spiro[1H-1,4-benzodiazepin- 3,4'-piperidin]-2(3H)-on

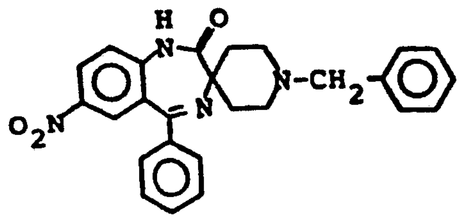 205 15

27 7-Chlor-5-(2-furyl)-1'-benzyl-spiro[1H-1,4-benzodiazepin- 3,4'-piperidin]-2(3H)-on

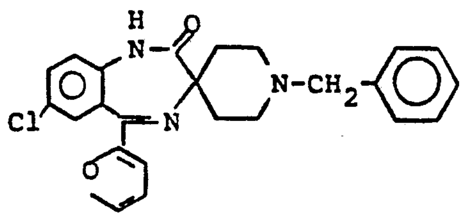 226-227 81

28 7-Chlor-5-(2-thiazolyl)-1'-benzyl-spiro[1H-1,4-benzodiazepin- 3,4'-piperidin]-2(3H)-on

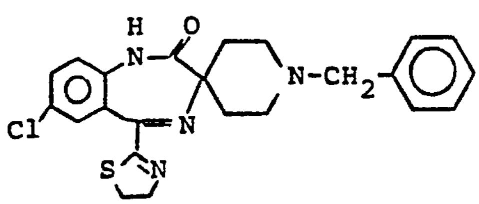 214-215 82

29 7-Chlor-5-methyl-1'-benzyl-spiro[1H-1,4-benzodiazepin- 3,4'-piperidin]-2(3H)-on

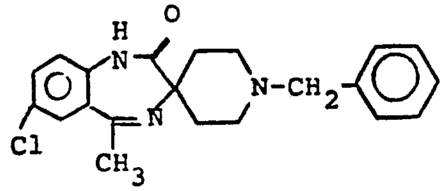 221-222 48

30 7-Brom-5-phenyl-1'-benzyl-spiro[1H-1,4-benzodiazepin- 3,4'-piperidin]-2(3H)-on

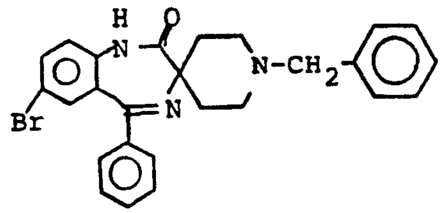 227-228 54

31 7-Trifluormethyl-5-phenyl-1'-benzyl-spiro[1H-1,4-benzodiazepin- 3,4'-piperidin]-2(3H)-on

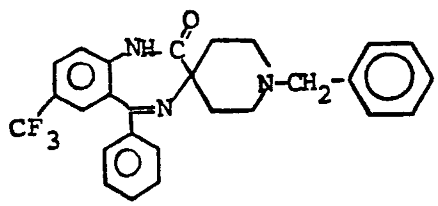 173 60

32 7-Chlor-5-(3-jodphenyl)-1'-benzyl-spiro[1H-1,4-benzodiazepin- 3,4'-piperidin]-2(3H)-on

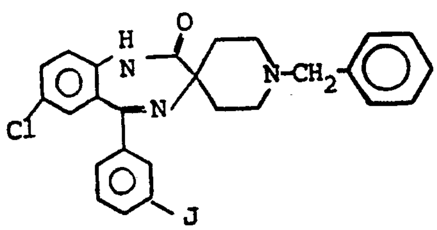 232 74

**Beispiel 33**

**5-Methyl-spiro[1H-1,4-benzodiazepin-3,4'-piperidin]-2(3H)-on**

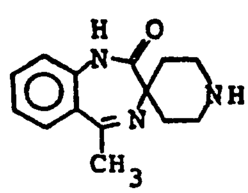

6,0 g (0,016 Mol) 5-Methyl-1'-benzyloxycarbonyl-spiro[1H-1,4-benzodiazepin-3,4'-piperidin]-2(3H)-on werden in 250 ml Ethanol in Gegenwart von 0,5 g Palladiumkohle bei 20°C hydriert. Nach dem Abfiltrieren vom Katalysator engt man das Filtrat ein und kristallisiert den Rückstand durch Zugabe von Diethylether.

Ausbeute: 3,5 g, entsprechend 90 % der Theorie; Fp. 220 - 222°C.

**Beispiel 34**

**7-Chlor-5-(4-chlorphenyl)-spiro[1H-1,4-benzodiazepin-3,4'-piperidin]-2(3H)-on**

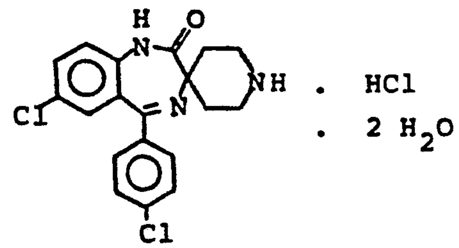

8,8 g (0,017 Mol) 5-(4-Chlorphenyl)-7-chlor-1'-benzyloxycarbonyl-spiro[1H-1,4-benzodiazepin-3,4'-piperidin]-2(3H)-on werden in 150 ml Petrolether (Siedebreich 60 - 80°C) suspendiert und bei 20°C unter Rühren mit 10 ml 38-proz. HBr-Eisessig-Lösung versetzt. Man rührt die Reaktionsmischung 6 Stunden bei Raumtemperatur, saugt das kristalline Reaktionsprodukt ab und wäscht mit Petrolether nach. Das Produkt wird in Wasser gelöst, die wäßrige Lösung mit Diethylether geschüttelt und mit konzentrierter wäßriger Ammoniaklösung alkalisiert. Die sich ausscheidende Base wird in Methylenchlorid aufgenommen, die organische Phase wird über $Na_2SO_4$ getrocknet und filtriert. Nach Abdampfen des Lösungsmittels erhält man die Base in Form hellgelber Kristalle.
Ausbeute: 5,2 g, entsprechend 80 % der Theorie; Fp. 204 - 206°C.

Hydrochlorid:
5,2 g (0,014 Mol) der erhaltenen Base werden mit 14 ml 1 n Salzsäure versetzt und die Lösung im Vakuum eingedampft. Man erhält das 5-(4-Chlorphenyl)-7-chlor-spiro[1H-1,4-benzodiazepin-3,4'-piperidin]-2(3H)-on hydrochloriddihydrat.
Ausbeute: 5,9 g, entsprechend 95 % der Theorie;
Fp. > 250°C (Zers.).

**Analog zu Beispiel 34 wurden hergestellt:**

| Beisp. Nr. | Formel | Fp. (°C) | (Ausbeute) (% d.Th.) |
|---|---|---|---|
| 35 | 5-Phenyl-spiro[1H-1,4-benzodiazepin-3,4'-piperidin]-2(3H)-on | | |
| | 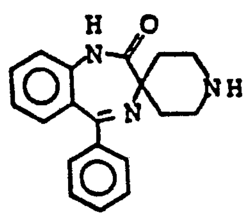 | 170 | 59 |
| 36 | 5-(2-Fluorphenyl)-spiro[1H-1,4-benzodiazepin-3,4'-piperidin]-2(3H)-on | | |
| | 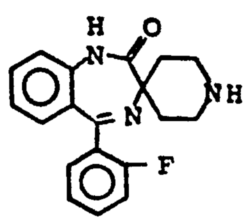 | 206 | 73 |
| 37 | 7-Chlor-5-(2-fluorphenyl)-spiro[1H-1,4-benzodiazepin- 3,4'-piperidin]-2(3H)-on | | |

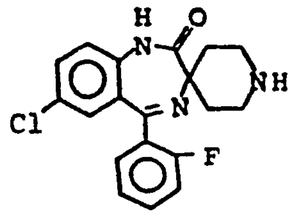 170 68

38 7-Chlor-5-(2-chlorphenyl)-spiro[1H-1,4-benzodiazepin- 3,4'-piperidin]-2(3H)-on

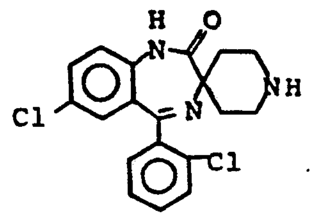 182 85

39 8-Methyl-5-phenyl-spiro[1H-1,4-benzodiazepin- 3,4'-piperidin]-2(3H)-on

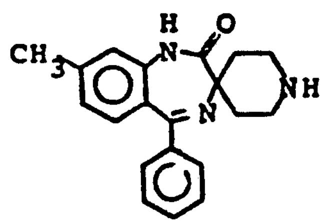 . HCl 260 97

40 7-Chlor-5-(2-thienyl)-spiro[1H-1,4-benzodiazepin- 3,4'-piperidin]-2(3H)-on

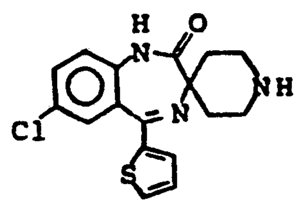 157-159 92

41 7-Chlor-5-(2-pyridyl)-spiro[1H-1,4-benzodiazepin- 3,4'-piperidin]-2(3H)-on

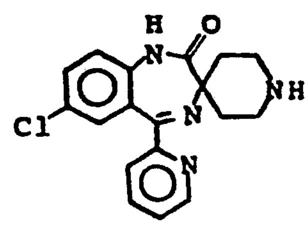 . 2 HCl >260 (Zers.) 73

42 7-Chlor-5-cyclohexyl-spiro[1H-1,4-benzodiazepin- 3,4'-piperidin]-2(3H)-on

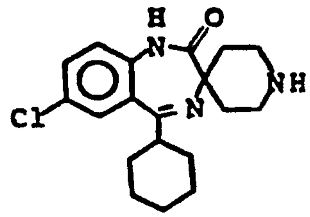 125 51

43 7-Chlor-5-isopropyl-spiro[1H-1,4-benzodiazepin- 3,4'-piperidin]-2(3H)-on

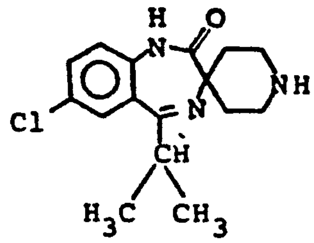 . HCl . $H_2O$ 96

ab 150° Zers.

44 7-Chlor-5-butyl-spiro[1H-1,4-benzodiazepin- 3,4'-piperidin]-2(3H)-on

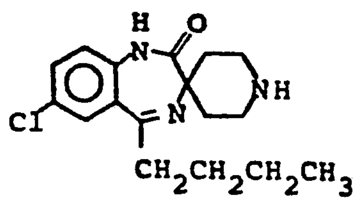 127-128 80

45 5-(4-Methylphenyl)-spiro[1H-1,4-benzodiazepin-3,4'-piperidin]-2(3H)-on

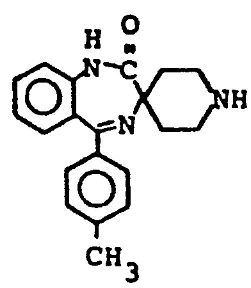 214-216 93

46 7-Nitro-5-phenyl-spiro[1H-1,4-benzodiazepin-3,4'-piperidin]-2(3H)-on

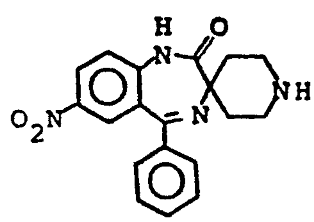 . HCl 260 97

47 7-Chlor-5-(2-furyl)-spiro[1H-1,4-benzodiazepin-3,4'-piperidin]-2(3H)-on

| | | | |
|---|---|---|---|
| | 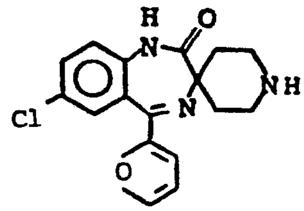 | 159-160 (Zers.) | 86 |
| 48 | 7-Chlor-5-(2-thiazolyl)-spiro[1H-1,4-benzodiazepin- 3,4'-piperidin]-2(3H)-on | | |
| | 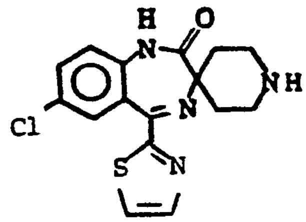 | 140 | 85 |
| 49 | 7-Chlor-5-methyl-spiro[1H-1,4-benzodiazepin-3,4'-piperidin]-2(3H)-on | | |
| | 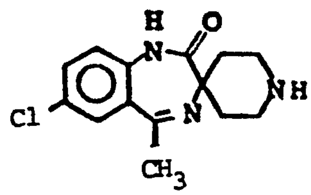 | 240 (Zers.) | 75 |
| 50 | 7-Brom-5-phenyl-spiro[1H-1,4-benzodiazepin-3,4-piperidin]-2(3H)-on | | |
| | 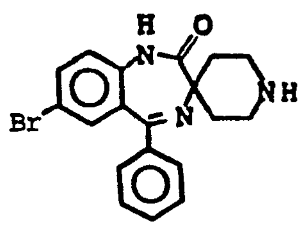 | 156 | 92 |
| 51 | 7-Trifluormethyl-5-phenyl-spiro[1H-1,4-benzodiazepin- 3,4-piperidin]-2(3H)-on | | |
| | 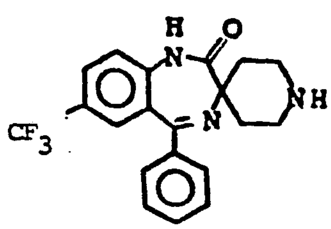 | 227 | 95 |
| 52 | 7-Chlor-5-(3-jodphenyl)-spiro[1H-1,4-benzodiazepin- 3,4-piperidin]-2(3H)-on | | |

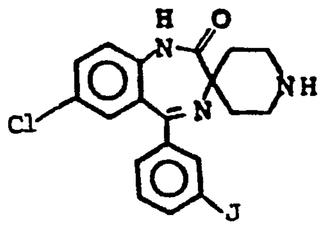 218 96

**Beispiel 53**

**7-Chlor-5-(4-chlorphenyl)-1'-benzyloxycarbonyl-spiro[1H-1,4-benzodiazepin-3,4'-piperidin]-2(3H)-on**

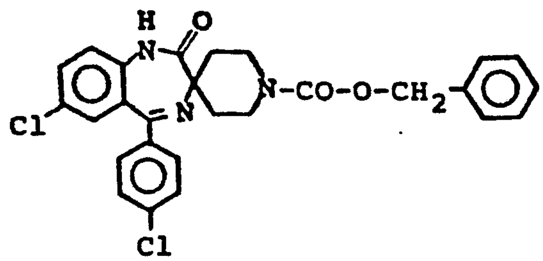

Zu einer Mischung aus 9,5 g (0,02 Mol) 7-Chlor-5-(4-chlorphenyl)-1'-benzyl-spiro[1H-1,4-benzodiazepin-3,4'-piperidin]-2(3H)-on und 5 g (0,06 Mol) $NaHCO_3$ in 150 ml Methylenchlorid werden 20 ml (0,06 Mol) eine 50-proz. Lösung von Chlorameisensäurebenzylester in Toluol gegeben. Das Reaktionsgemisch wird 3 Stunden unter Rückflußkühlung gekocht. Anschließend gießt man auf Eis, trennt die organische Phase ab und wäscht zweimal mit 10-proz. $NaHCO_3$-Lösung. Nach dem Trocknen über $Na_2SO_4$ wird filtriert, und das Lösungsmittel abgedampft. Der ölige Rückstand wird mit Petrolether zur Kristallisation gebracht. Die Verbindung wird abgesaugt, mit n-Hexan ausgekocht und im Vakuum bei 60° C getrocknet.

Ausbeute: 8,8 g, entsprechend 83 % der Theorie; Fp. 130° C.

**Analog zu Beispiel 53 wurden hergestellt:**

| Beisp. Nr. | Formel | Fp. (° C) | (Ausbeute) (% d.Th.) |
|---|---|---|---|
| 54 | 5-Phenyl-1'-benzyloxycarbonyl-spiro[1H-1,4-benzodiazepin- 3,4'-piperidin]-2(3H)-on | | |
| | 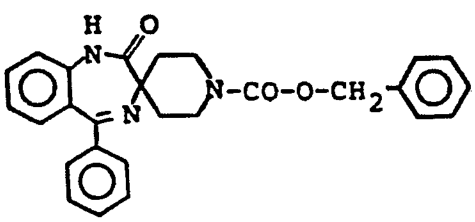 | 196 | 90 |
| 55 | 5-(2-Fluorphenyl)-1'-benzyloxycarbonyl-spiro[1H-1,4-benzodiazepin- 3,4'-piperidin]-2-(3H)-on | | |

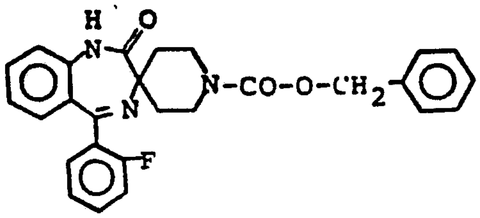 178 87

56 5-(2-Fluorphenyl)-7-chlor-1'-benzyloxycarbonyl-spiro[1H-1,4-benzodiazepin- 3,4'-piperidin]-2(3H)-on

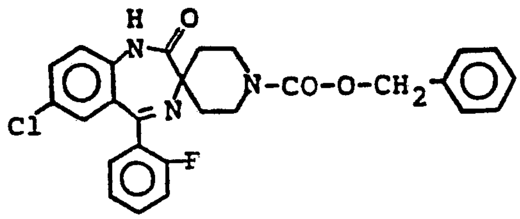 173 68

57 5-(2-Chlorphenyl)-7-chlor-1'-benzyloxycarbonyl- spiro[1H-1,4-benzodiazepin-3,4'-piperidin]-2(3H)-on

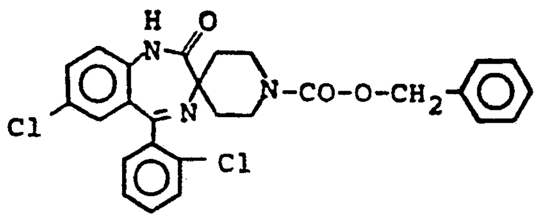 166 93

58 8-Methyl-1'-benzyloxycarbonyl-spiro[1H-1,4-benzodiazepin- 3,4'-piperidin]-2(3H)-on

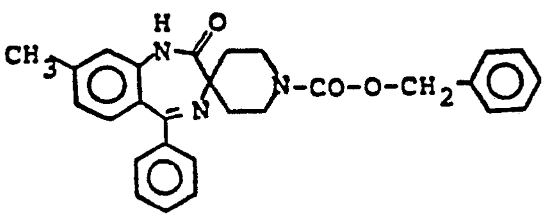 187 73

59 7-Chlor-5-(2-thienyl)-1'-benzyloxycarbonyl-spiro[1H- 1,4-benzodiazepin-3,4'-piperidin]-2(3H)-on

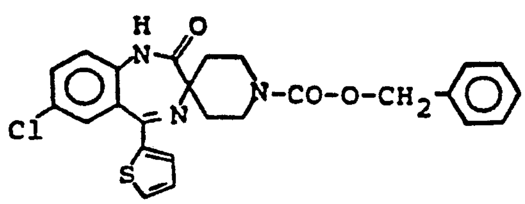 180-181 57

60 7-Chlor-5-(2-pyridyl)-1'-benzyloxycarbonyl-spiro[1H- 1,4-benzodiazepin-3,4'-piperidin]-2(3H)-on

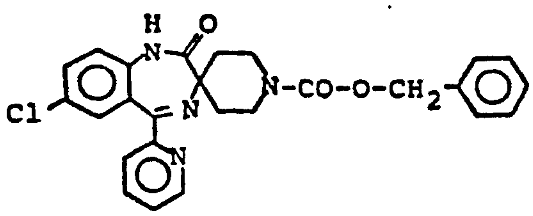

213-214 91

61 7-Chlor-5-cyclohexyl-1'-benzyloxycarbonyl-spiro[1H- 1,4-benzodiazepin-3,4'-piperidin]-2(3H)-on

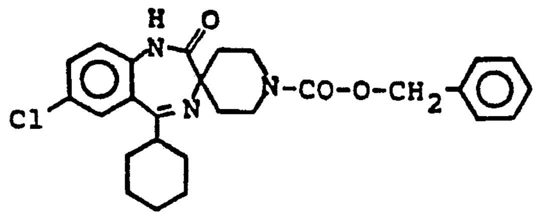

239-240 91

62 5-Methyl-1'-benzyloxycarbonyl-spiro[1H-1,4-benzodiazepin- 3,4'-piperidin]-2(3H)-on

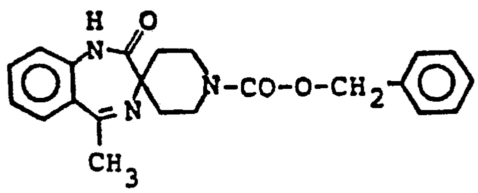

159-160 95

63 7-Chlor-5-isopropyl-1'-benzyloxycarbonyl-spiro[1H- 1,4-benzodiazepin-3,4'-piperidin]-2(3H)-on

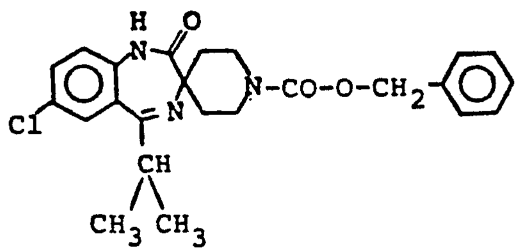

215-218 72

64 7-Chlor-5-butyl-1'-benzyloxycarbonyl-spiro[1H-1,4- benzodiazepin-3,4'-piperidin]-2(3H)-on

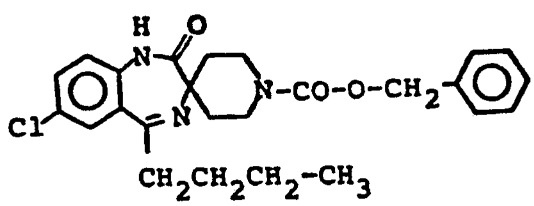

176-177 85

65 5-(4-Methylphenyl)-1'-benzyloxycarbonyl-spiro[1H- 1,4-benzodiazepin-3,4'-piperidin]-2(3H)-on

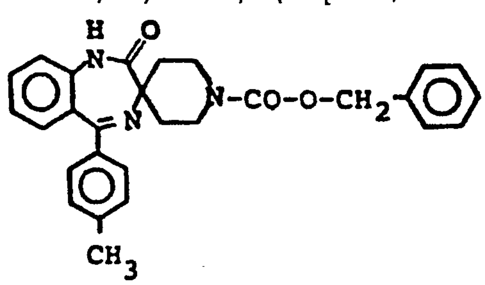

149 62

66 7-Nitro-5-phenyl-1'-benzyloxycarbonyl-spiro[1H-1,4- benzodiazepin-3,4'-piperidin]-2(3H)-on

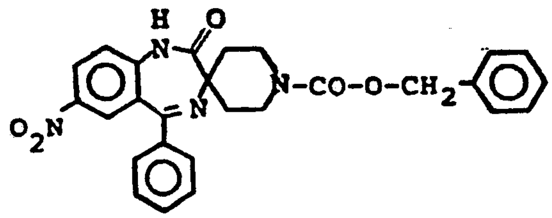 135 94

67 7-Chlor-5-(2-furyl)-1'-benzyloxycarbonyl-spiro[1H- 1,4-benzodiazepin-3,4'-piperidin]-2(3H)-on

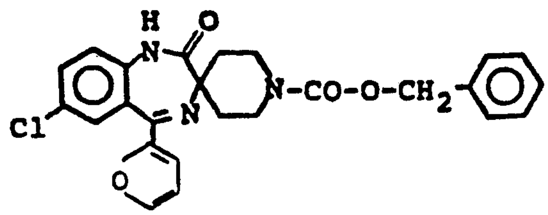 176-177 85

68 7-Chlor-5-(2-thiazolyl)-1'-benzyloxycarbonyl-spiro[1H- 1,4-benzodiazepin-3,4'-piperidin]-2(3H)-on

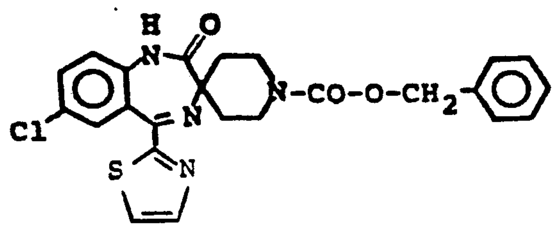 215-216 87

69 7-Chlor-5-methyl-1'-benzyloxycarbonyl-spiro[1H- 1,4-benzodiazepin-3,4'-piperidin]-2(3H)-on

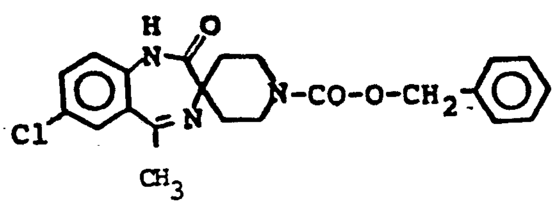 147-148 98

70 7-Brom-5-phenyl-1'-benzyloxycarbonyl-spiro[1H- 1,4-benzodiazepin-3,4'-piperidin]-2(3H)-on

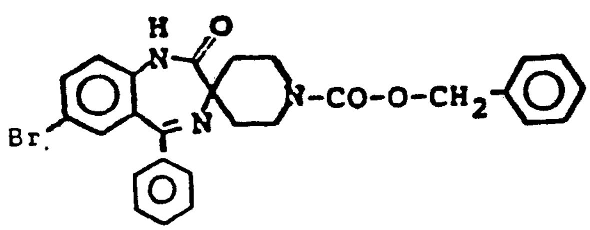 203-204 88

71 7-Trifluormethyl-5-phenyl-1'-benzyloxycarbonyl- spiro[1H-1,4-benzodiazepin-3,4'-piperidin]-2(3H)-on

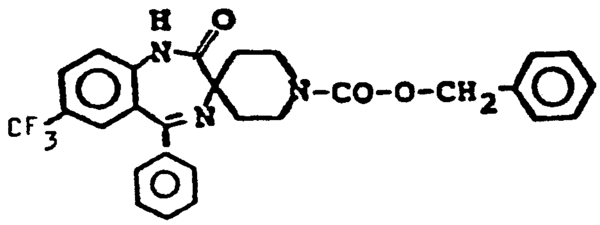 100 42

72 7-Chlor-5-(3-jodphenyl)-1'-benzyloxycarbonyl- spiro[1H-1,4-benzodiazepin-3,4'-piperidin]-2(3H)-on

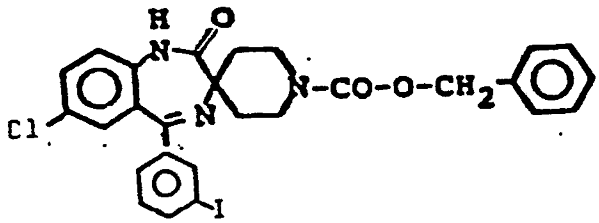 179 74

**Herstellung von Aniliden als Zwischenprodukte:**

Die Herstellung der Anilide erfolgt analog zu den Beispielen 2 und 14. Die Anilide werden als Rohprodukte ohne weitere Reinigung direkt weiterverarbeitet.

In den folgenden Beispielen werden einige der Anilide beschrieben:

| Beisp.-Nr. | Aminoketon | Anilid | Fp (°C) | Ausbeute (% d.Th.) |
|---|---|---|---|---|
| 73 | 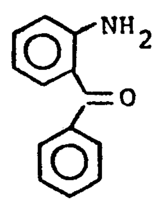 | 1-Benzyl-4-amino-4-piperidino-carbonsäure-2'-benzoylanilid 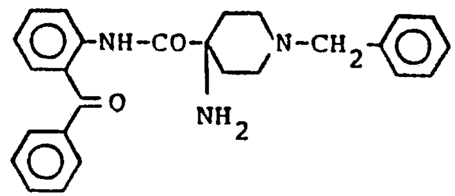 | Öl | ~80 |
| 74 | 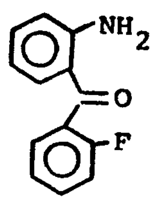 | 1-Benzyl-4-amino-4-piperidino-carbonsäure-2'-(2-fluorbenzoyl)-anilid 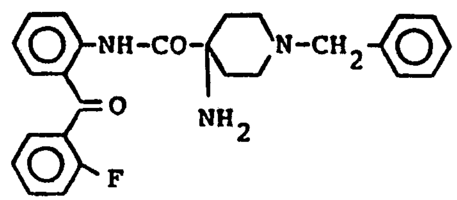 | Öl | ~53 |

| Beisp.-Nr. | Aminoketon | Anilid | Fp(°C) | Ausbeute (% d.T |
|---|---|---|---|---|
| 75 | 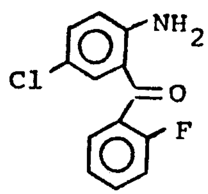 | 1-Benzyl-4-amino-4-piperidino-carbonsäure-2'-(2-fluorbenzoyl)-4'-chloranilid 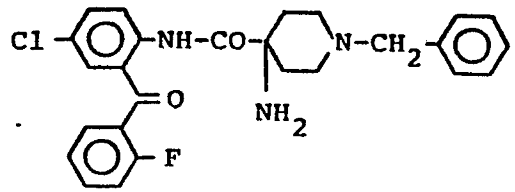 | 135-8 | ~45 |
| 76 | 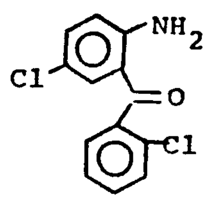 | 1-Benzyl-4-amino-4-piperidino-carbonsäure-2'-(2-chlorbenzoyl)-4'-chloranilid 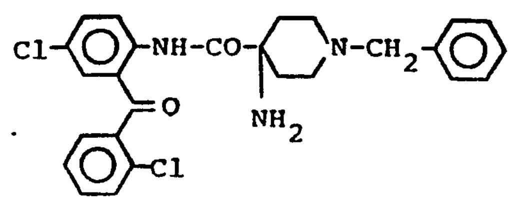 | Öl | ~38 |

| Beisp.-Nr. | Aminoketon | Anilid | Fp. (°C) | Ausbeute (% d.Th |
|---|---|---|---|---|
| 77 | 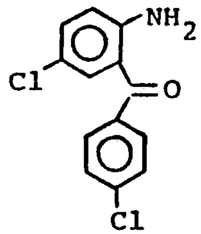<br>L.H. Sternbach et al.<br>J. Org. Chem. 26, 4488 (1962) | 1-Benzyl-4-amino-4-piperidino-carbonsäure-2'-(4-chlorbenzoyl)-4'-chloranilid<br>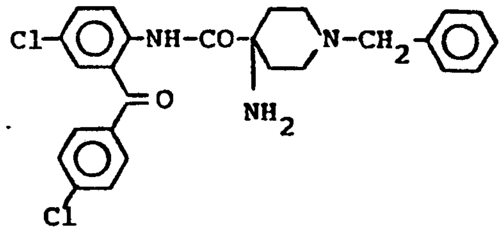 | 81 | 51 |
| 78 | 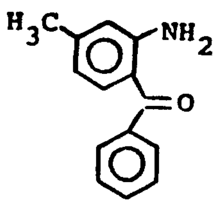 | 1-Benzyl-4-amino-4-piperidino-carbonsäure-2'-benzoyl-5'-methyl-anilid<br>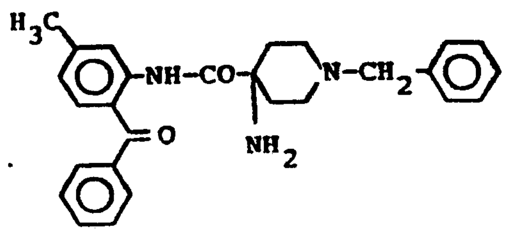 | 81 | 40 |

| Beisp.-Nr. | Aminoketon | Anilid | Fp. (°C) | Ausbeute (% d.T |
|---|---|---|---|---|
| 79 | 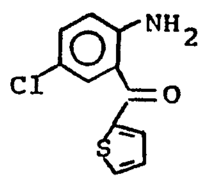 F.Hunziker et al. Eur.J.Med.Chem.16, 391 (1981) | 1-Benzyl-4-amino-4-piperidino-carbonsäure-2'-(2-thienoyl)-4'-chloranilid 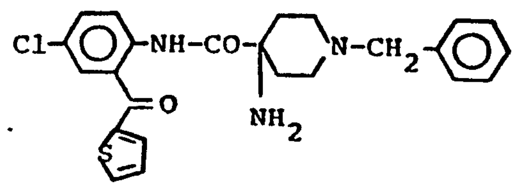 | Öl | 42 |
| 80 | 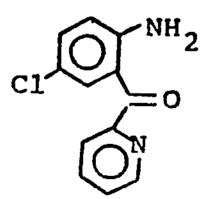 R.I.Fryer et al. J.Pharm.Sci. 53, 264 (1964) | 1-Benzyl-4-amino-4-piperidino-carbonsäure-2'-(2-pyridocarbonyl)-4'-chloranilid 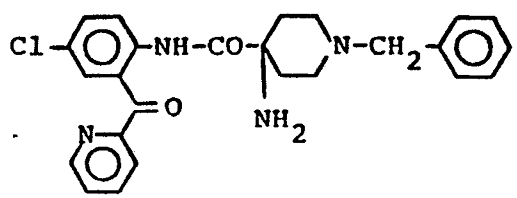 | Öl | 41 |

| Beisp.-Nr. | Aminoketon | Anilid | Fp.(°C) | Ausbeute (% d.Th |
|---|---|---|---|---|
| 81 | 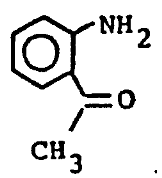 | 1-Benzyl-4-amino-4-piperidino-carbonsäure-2'-acetylanilid 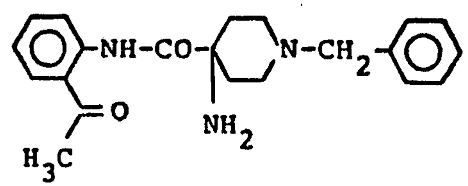 | Öl | 38 |
| 82 | 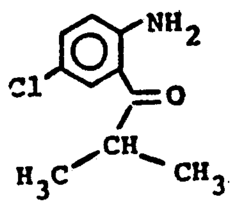 FR 1 391 752 (CA 63, 4361 (1965)) | 1-Benzyl-4-amino-4-piperidino-carbonsäure-2'-(2-methylbutyryl)-4'-chloranilid 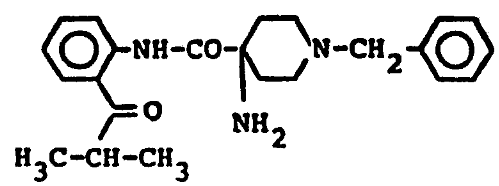 | Öl | 56 |

| Beisp.-Nr. | Aminoketon | Anilid | Fp. (°C) | Ausbeute (% d.Th |
|---|---|---|---|---|
| 83 | 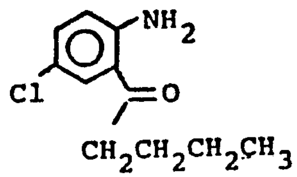 FR 1 391 752 (CA 63, 4361 (1965)) | 1-Benzyl-4-amino-4-piperidino-carbonsäure-2'-valeroyl-4'-chlor-anilid 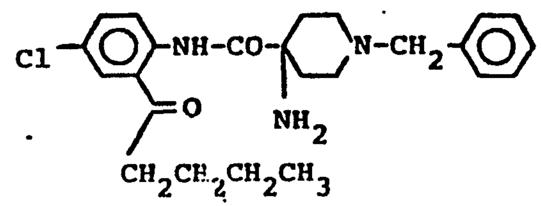 | 100-103 | 61 |
| 84 | 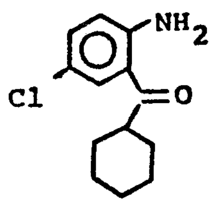 S.C.Bell et al. J.Med.Pharm.Chem. 5, 63 (1962) | 1-Benzyl-4-amino-4-piperidino-carbonsäure-2'-cyclohexanoyl-4'-chloranilid 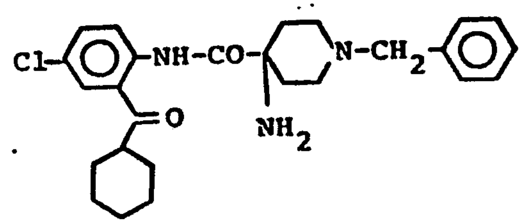 | 164 | 40 |

| Beisp.-Nr. | Aminoketon | Anilid | Fp. (°C) | Ausbeute (% d.Th.) |
|---|---|---|---|---|
| 85 | 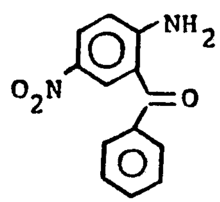 | 1-Benzyl-4-amino-4-piperidino-carbonsäure-2'-benzoyl-4'-nitro-anilid 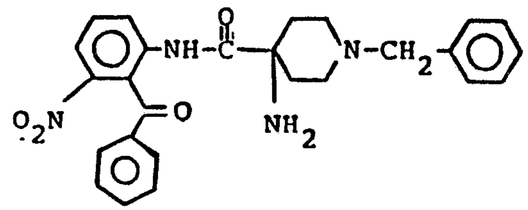 | ö1 | 34 |
| 86 | 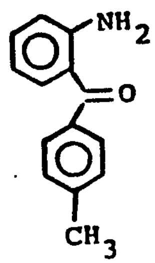 | 1-Benzyl-4-amino-4-piperidino-carbonsäure-2'-(4-methylbenzoyl)-anilid 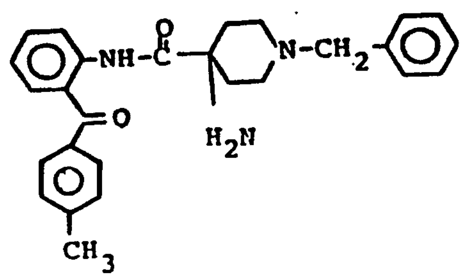 | ö1 | 38 |

| Bsp.-Nr. | Aminoketon | Anilid | Fp. (°C) | Ausbeute (% d. Th.) |
|---|---|---|---|---|
| 87 | 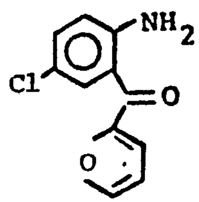 | 1-Benzyl-4-amino-4-piperidinocarbonsäure-2'-(2-furanoyl)-4'-chloranilid 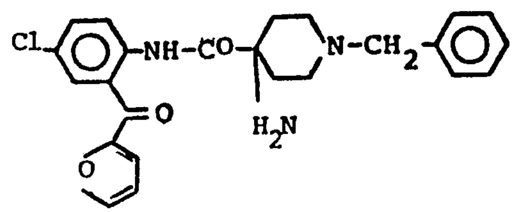 | 132-133 | 59 |
| 88 | 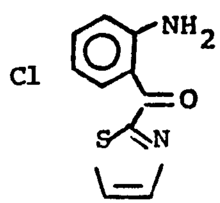 | 1-Benzyl-4-amino-4-piperidinocarbonsäure-2'-(2-thiazoloyl)-4'-chloranilid 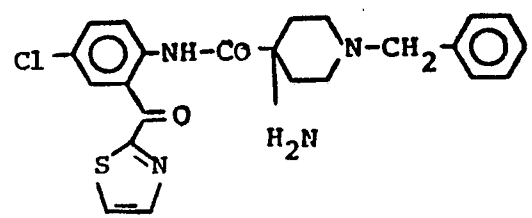 | 220-221 | 50 |

| Bsp.-Nr. | Aminoketon | Anilid | Fp. (°C) | Ausbeute (% d. Th.) |
|---|---|---|---|---|
| 89 | 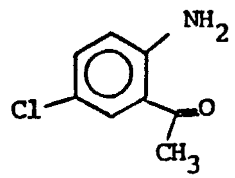 | 1-Benzyl-4-amino-4-piperidincarbonsäure-2'-acetyl-4'-chloranilid 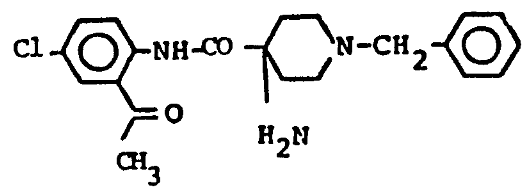 | 251-252 | 50 |
| 90 | 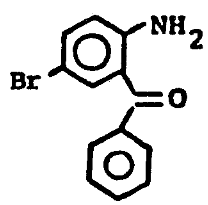 | 1-Benzyl-4-amino-4-piperidinocarbonsäure-2'-phenyl4'-bromanilid 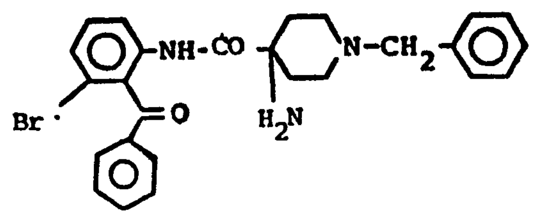 | 81 | 50 |

| Bsp.-Nr. | Aminoketon | Anilid | Fp. (°C) | Ausbeute (% d. Th.) |
|---|---|---|---|---|
| 91 | 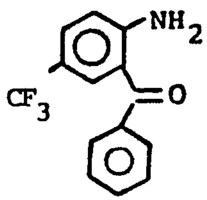 | 1-benzyl-4-amino-4-piperidinocarbonsäure-2'-phenyl-4'-trifluormethylanilid 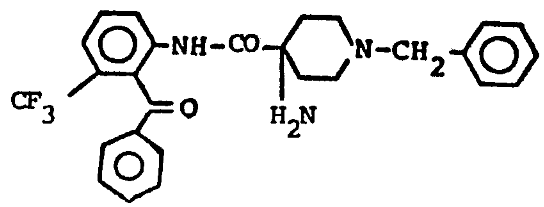 | Öl | 51 |
| 92 | 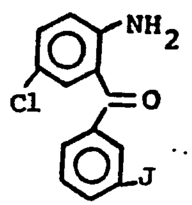 | 1-Benzyl-4-amino-4-piperidinocarbonsäure-2'-(3-jodphenyl)-4'-chloranilid 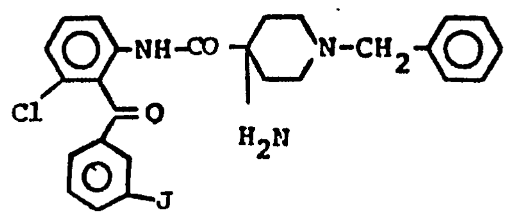 | 75-80 | 56 |

**Patentansprüche**

1. Benzodiazepine der Formel

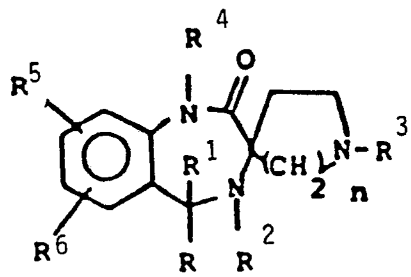

in welcher

R für H, gesättigtes oder eine Doppelbindung enthaltendes, geradkettiges, verzweigtes oder cyclisches Alkyl mit 1 bis 8 Kohlenstoffatomen, für einen Aralkylrest mit einer Alkyl- oder Alkylenkette von 1 bis 3 C-Atomen und einem unsubstituierten oder gegebenenfalls mit ein oder zwei Substituenten aus der Reihe Cl, Br, F, CN, $CF_3$, $NO_2$, $CH_3$, $C_2H_5$ oder $OCH_3$ versehenen Phenylrest,

für einen gegebenenfalls mit ein oder zwei Substituenten aus der Reihe Cl, F, Br, CN, $CF_3$, $NO_2$, $CH_3$, $C_2H_5$ oder $OCH_3$ versehenen Phenylrest oder einen 5- oder 6-gliedrigen aromatischen Rest mit 1 oder 2 Heteroatomen aus der Reihe O, N, S steht,

$R_1$ für H steht,

$R_2$, $R_3$ und $R_4$ unabhängig voneinander für H, einen Alkylrest mit 1 bis 6 Kohlenstoffatomen, oder für einen Aralkylrest mit einer Alkyl- oder Alkylenkette von 1 bis 3 C-Atomen und einem unsubstituierten oder gegebenenfalls mit einem oder zwei Substituenten aus der Reihe Cl, Br, F, CN, $CF_3$, $NO_2$, $CH_3$, $C_2H_5$ oder $CH_3$ versehenen Phenylrest stehen und gegebenenfalls

wobei

$R_1$ und $R_2$ auch gemeinsam eine Bindung bilden können,

$R_5$ und $R_6$ unabhängig voneinander für H, F, Cl, Br, CN, $CF_3$, $NO_2$, $CH_3$, $C_2H_5$ oder $OCH_3$ stehen und n für eine ganze Zahl von 1 bis 3 steht.

2. Benzodiazepine und Anspruch 1, in denen

R für einen unsubstituierten oder gegebenenfalls mit 1 oder 2 Substituenten aus der Reihe Cl, F, Br, CN, $CF_3$ oder $NO_2$ versehenen Phenylrest, oder einen 5- oder 6-gliedrigen heteroaromattischen Rest mit 1 oder 2 Heteroatomen aus der Reihe O, N, S steht,

$R_1$ für H steht,

$R_2$, $R_3$ und $R_4$ unabhängig voneinander für H oder einen Alkylrest mit 1 bis 4 Kohlenstoffatomen stehen und wobei

$R_1$ und $R_2$ auch gemeinsam eine Bindung bilden können,

$R_5$ und $R_6$ unabhängig voneinander für H, F, Cl, Br, CN, $CF_3$ oder $NO_2$ stehen und

n für 2 steht.

3. Benzodiazepin nach den Ansprüchen 1 und 2 der Formel

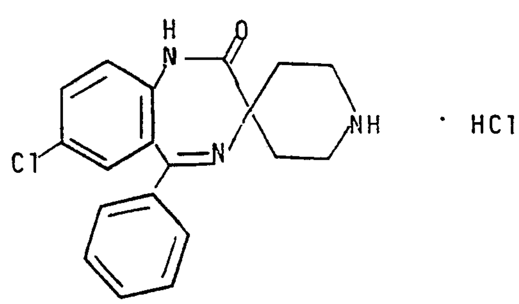

4. Benzodiazepine der Formel

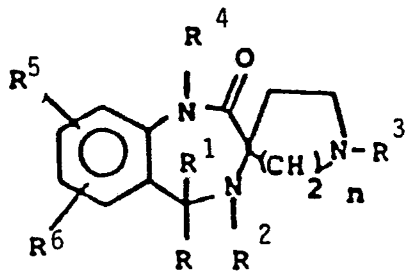

in welcher

R für H, gesättigtes oder eine Doppelbindung enthaltendes, geradkettiges, verzweigtes oder cyclisches Alkyl mit 1 bis 8 Kohlenstoffatomen, für einen Aralkylrest mit einer Alkyl- oder Alkylenkette von 1 bis 3 C-Atomen und einem i unsubstituierten oder gegebenenfalls mit ein oder zwei Substituenten aus der Reihe Cl, Br, F, CN, $CF_3$, $NO_2$, $CH_3$, $C_2H_5$ oder $OCH_3$ versehenen Phenylrest,

für einen gegebenenfalls mit ein oder zwei Substituenten aus der Reihe Cl, F, Br, CN, $CF_3$, $NO_2$, $CH_3$, $C_2H_5$ oder $OCH_3$ versehenen Phenylrest oder einen 5- oder 6-gliedrigen aromatischen Rest mit 1 oder 2 Heteroatomen aus der Reihe O, N, S steht.

$R_1$ für H steht,

$R_2$, $R_3$ und $R_4$ unabhängig voneinander für H, einen Alkylrest mit 1 bis 6 Kohlenstoffatomen, oder für einen Aralkylrest mit einer Alkyl- oder Alkylenkette von 1 bis 3 C-Atomen und einem unsubstituierten oder gegebenenfalls mit einem oder zwei Substituenten aus der Reihe Cl, Br, F, CN, $CF_3$, $NO_2$, $CH_3$ $C_2H_5$ oder $CH_3$ versehenen Phenylrest stehen und gegebenenfalls

wobei

$R_1$ und $R_2$ auch gemeinsam eine Bindung bilden können,

$R_5$ und $R_6$ unabhängig voneinander für H, F, Cl, Br, CN, $CF_3$, $NO_2$, $CH_3$, $C_2H_5$ oder $OCH_3$ stehen und

n für eine ganze Zahl von 1 bis 3 steht,

zur therapeutischen Behandlung.

5. Verfahren zur Herstellung von Benzodiazepine der Formel

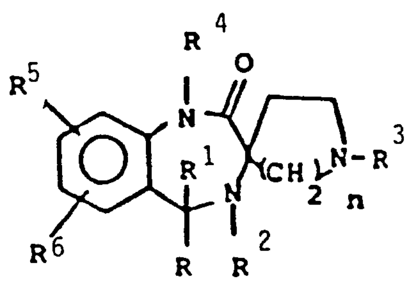

in welcher

R für H, gesättigtes oder eine Doppelbindung enthaltendes, geradkettiges, verzweigtes oder cyclisches Alkyl mit 1 bis 8 Kohlenstoffatomen, für einen Aralkylrest mit einer Alkyl- oder Alkylenkette von 1 bis 3 C-Atomen und einem unsubstituierten oder gegebenenfalls mit ein oder zwei Substituenten aus der Reihe Cl, Br, F, CN, $CF_3$, $NO_2$, $CH_3$, $C_2H_5$ oder $OCH_3$ versehenen Phenylrest,

für einen gegebenenfalls mit ein oder zwei Substituenten aus der Reihe Cl, F, Br, CN, $CF_3$, $NO_2$, $CH_3$, $C_2H_5$ oder $OCH_3$ versehenen Phenylrest oder einen 5- oder 6-gliedrigen aromatischen Rest mit 1 oder 2 Heteroatomen aus der Reihe O, N, S steht,

$R_1$ für H steht,

$R_2$, $R_3$ und $R_4$ unabhängig voneinander für H, einen Alkylrest mit 1 bis 6 Kohlenstoffatomen, oder für einen Aralkylrest mit einer Alkyl- oder Alkylenkette von 1 bis 3 C-Atomen und einem unsubstituierten oder gegebenenfalls mit einem oder zwei Substituenten aus der Reihe Cl, Br, F, CN, $CF_3$, $NO_2$, $CH_3$, $C_2H_5$ oder $CH_3$ versehenen Phenylrest stehen und gegebenenfalls

wobei

$R_5$ und $R_6$ auch gemeinsam eine Bindung bilden können,

$R_5$ und $R_6$ unabhängig voneinander für H, F, Cl, Br, CN, $CF_3$, $NO_2$, $CH_3$, $C_2H_5$ oder $OCH_3$ stehen und

n für eine ganze Zahl von 1 bis 3 steht,

dadurch gekennzeichnet, daß man Verbindungen der allgemeinen Formel

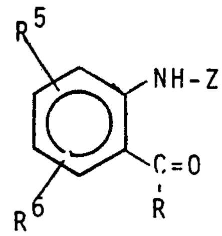

in der

$R_5$, $R_6$ und R die oben angegebene Bedeutung haben und

Z für H oder eine Schutzgruppe steht,

mit Verbindungen der allgemeinen Formel

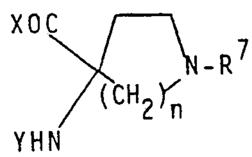

in welcher

COX für eine Carboxylfunktion oder eine aktivierte Carboxylfunktion, wie sie zur Knüpfung von Säureamidbindungen Verwendung findet, steht,

Y H oder eine in der Peptidchemie übliche Schutzgruppe bedeutet und

$R_7$ entweder die oben angegebene Bedeutung von $R_3$ hat oder für eine Schutzgruppe steht und

n die oben genannte Bedeutung hat,

umsetzt und in dem Fall, daß $R_7$ für eine Schutzgruppe steht, diese abspaltet

und gegebenenfalls die Reste $R_2$ und $R_4$ durch Alkylierung einführt.

6. Arzneimittel, enthaltend Benzodiazepine der Formel

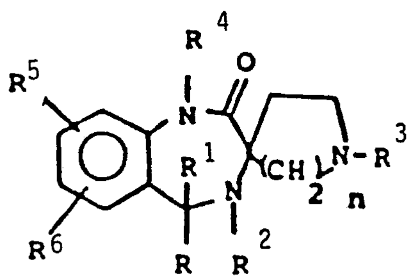

in welcher

R für H, gesättigtes oder eine Doppelbindung enthaltendes, geradkettiges, verzweigtes oder cyclisches Alkyl mit 1 bis 8 Kohlenstoffatomen, für einen Aralkylrest mit einer Alkyl- oder Alkylenkette von 1 bis 3 C-Atomen und einem unsubstituierten oder gegebenenfalls mit ein oder zwei Substituenten aus der Reihe Cl, Br, F, CN, $CF_3$, $NO_2$, $CH_3$, $C_2H_5$ oder $OCH_3$ versehenen Phenylrest,

für einen gegebenenfalls mit ein oder zwei Substituenten aus der Reihe Cl, F, Br, CN, $CF_3$, $NO_2$, $CH_3$, $C_2H_5$ oder $OCH_3$ versehenen Phenylrest oder einen 5- oder 6-gliedrigen aromatischen Rest mit 1 oder 2 Heteroatomen aus der Reihe O, N, S steht,

$R_1$ für H steht,

$R_2$, $R_3$ und $R_4$ unabhängig voneinander für H, einen Alkylrest mit 1 bis 6 Kohlenstoffatomen, oder für einen Aralkylrest mit einer Alkyl- oder Alkylenkette von 1 bis 3 C-Atomen und einem unsubstituierten oder gegebenenfalls mit einem oder zwei Substituenten aus der Reihe Cl, Br, F, CN, $CF_3$, $NO_2$, $CH_3$, $C_2H_5$ oder $CH_3$ versehenen Phenylrest stehen und gegebenenfalls

wobei

$R_1$ und $R_2$ auch gemeinsam eine Bindung bilden können,

$R_5$ und $R_6$ unabhängig voneinander für H, F, Cl, Br, CN, $CF_3$, $NO_2$, $CH_3$, $C_2H_5$ oder $OCH_3$ stehen und

n für eine ganze Zahl von 1 bis 3 steht.

7. Arzneimittel nach Anspruch 6, enthaltend 0,1 bis 99,5 Gew.-% des Benzodiazepins.

8. Verwendung von Benzodiazepine der Formel

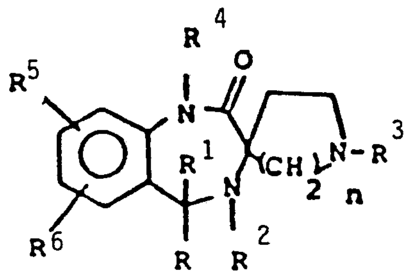

in welcher

R für H, gesättigtes oder eine Doppelbindung enthaltendes, geradkettiges, verzweigtes oder cyclisches Alkyl mit 1 bis 8 Kohlenstoffatomen, für einen Aralkylrest mit einer Alkyl- oder Alkylenkette von 1 bis 3 C-Atomen und einem unsubstituierten oder gegebenenfalls mit ein oder zwei Substituenten aus der Reihe Cl, Br, F, CN, $CF_3$, $NO_2$, $CH_3$, $C_2H_5$ oder $OCH_3$ versehenen Phenylrest,

für einen gegebenenfalls mit ein oder zwei Substituenten aus der Reihe Cl, F, Br, CN, $CF_3$, $NO_2$, $CH_3$, $C_2H_5$ oder $OCH_3$ versehenen Phenylrest oder einen 5- oder 6-gliedrigen aromatischen Rest mit 1 oder 2 Heteroatomen aus der Reihe O, N, S steht,

$R_1$ für H steht,

$R_2$, $R_3$ und $R_4$ unabhängig voneinander für H, einen Alkylrest mit 1 bis 6 Kohlenstoffatomen, oder für einen Aralkylrest mit einer Alkyl- oder Alkylenkette von 1 bis 3 C-Atomen und einem unsubstituierten oder gegebenenfalls mit einem oder zwei Substituenten aus der Reihe Cl, Br, F, CN, $CF_3$, $NO_2$, $CH_3$, $C_2H_5$ oder $CH_3$ versehenen Phenylrest stehen und gegebenenfalls

wobei

$R_1$ und $R_2$ auch gemeinsam eine Bindung bilden können,

$R_5$ und $R_6$ unabhängig voneinander für H, F, Cl, Br, CN, $CF_3$, $NO_2$, $CH_3$, $C_2H_5$ oder $OCH_3$ stehen und

n für eine ganze Zahl von 1 bis 3 steht

zur Herstellung von Arzneimitteln.

9. Verwendung nach Anspruch 8 zur Herstellung von Nootropika.

10. Verwendung nach Anspruch 8 zur Herstellung von Arzneimittel zur Behandlung von altersbedingten Störungen der Gehirnfunktion.

11. Verwendung nach Anspruch 8 zur Herstellung von Arzneimittel zur Behandlung von Amnesien.

**Claims**

1. Benzodiazepines of the formula

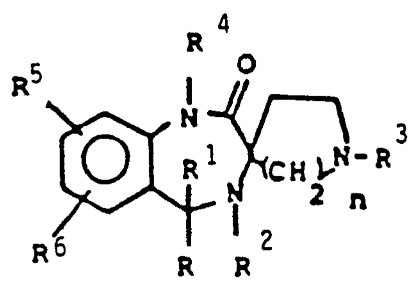

in which

R represents H, straight-chain, branched or cyclic alkyl having 1 to 8 carbon atoms which is saturated or contains one double bond, an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $OCH_3$,

represents a phenyl radical which optionally contains one or two substituents from the series comprising Cl, F, Br, CN, $CF_3$, $NO_2$, $CH_3$, $C_2H_5$ or $OCH_3$, or represents a 5- or 6-membered aromatic radical having 1 or 2 heteroatoms from the series comprising O, N and S,

$R_1$ represents H,

$R_2$, $R_3$ and $R_4$, independently of one another, represent H, an alkyl radical having 1 to 6 carbon atoms, or an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $CH_3$, and, where appropriate,

$R_1$ and $R_2$ together can also form a bond,
$R_5$ and $R_6$, independently of one another, represents H, F, Cl, Br, CN, $CF_3$, $NO_2$, $CH_3$, $C_2H_5$ or $OCH_3$, and
n represents an integer from 1 to 3.

2. Benzodiazepines according to Claim 1, in which
R represents a phenyl radical which is unsubstituted or optionally contains 1 or 2 substituents from the series comprising Cl, F, Br, CN, $CF_3$ or $NO_2$, or represents a 5- or 6-membered heteroaromatic radical having 1 or 2 heteroatoms from the series comprising O, N and S,
$R_1$ represents H,
$R_2$, $R_3$ and $R_4$, independently of one another, represent H or an alkyl radical having 1 to 4 carbon atoms, and
$R_1$ and $R_2$ together can also form a bond,
$R_5$ and $R_6$, independently of one another, represent H, F, Cl, Br, CN, $CF_3$ or $NO_2$, and
n represents 2.

3. Benzodiazepine according to Claims 1 and 2, of the formula

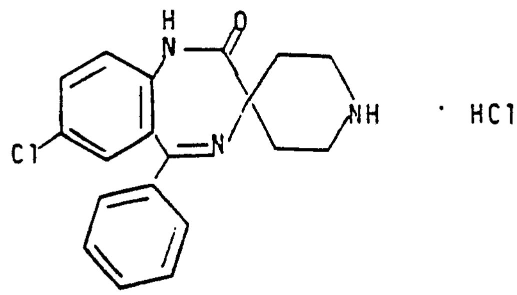

4. Benzodiazepines of the formula

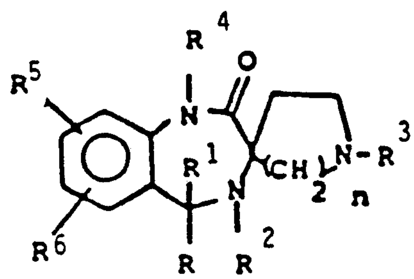

in which
R represents H, straight-chain, branched or cyclic alkyl having 1 to 8 carbon atoms which is saturated or contains one double bond, an aralkyl radical having an alkyl or alkylene chain or 1 to 3 C atoms and a phenyl radical which is unsubstituted or optionally contains one or two substituents from the series comprising Cl, Br, F, CN, $CF_3$, $NO_2$, $CH_3$, $C_2H_5$ or $OCH_3$,
represents a phenyl radical which optionally contains one or two substituents from the series comprising Cl, F, Br, CN, $CF_3$, $NO_2$, $CH_3$, $C_2H_5$ or $OCH_3$, or represents a 5- or 6-membered aromatic radical having 1 or 2 heteroatoms from the series comprising O, N and S,
$R_1$ represents H,
$R_2$, $R_3$ and $R_4$, independently of one another, represent H, an alkyl radical having 1 to 6 carbon atoms, or an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $OCH_3$, and, where appropriate,
$R_1$ and $R_2$ together can also form a bond
$R_5$ and $R_6$, independently of one another, represent H, F, Cl, Br, CN, $CF_3$, $NO_2$, $CH_3$, $C_2H_5$ or $OCH_3$, and
n represents an integer from 1 to 3,
for therapeutic treatment.

5. Process for the preparation of benzodiazepines of the formula

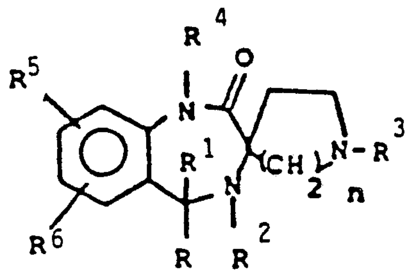

in which

R represents H, straight-chain, branched or cyclic alkyl having 1 to 8 carbon atoms which is saturated or contains one double bond, an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $OCH_3$,

represents a phenyl radical which optionally contains one or two substituents from the series comprising Cl, F, Br, CN, $CF_3$, $NO_2$, $CH_3$, $C_2H_5$ or $OCH_3$, or represents a 5- or 6-membered aromatic radical having 1 or 2 heteroatoms from the series comprising O, N and S,

$R_1$ represents H,

$R_2$, $R_3$ and $R_4$, independently of one another, represent H, an alkyl radical having 1 to 6 carbon atoms, or an aralkyl radical having an alkyl or alkylene chain of 1 to 3 C atoms and a phenyl radical which is unsubstituted or optionally contains 1 or 2 substituents from the series comprising Cl, Br, F, CN, $CF_3$, $NO_2$, $CH_3$, $C_2H_5$ or $CH_3$, and, where appropriate,

$R_1$ and $R_2$ together can also form a bond,

$R_5$ and $R_6$, independently of one another, represent H, F, Cl, Br, CN, $CF_3$, $NO_2$, $CH_3$, $C_2H_5$ or $OCH_3$, and

n represents an integer from 1 to 3,

characterized in that the compounds of the general formula

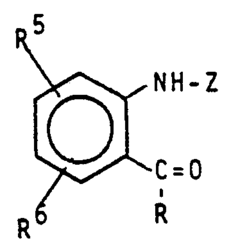

in which

$R_5$, $R_6$ and R have the abovementioned meaning and

Z represents H or a protecting group, are reacted with compounds of the general formula

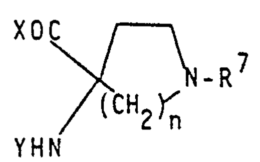

in which

COX represents a carboxyl function or an activated carboxyl function, as used for linking acid amide bonds,

Y denotes H or a protecting group which is customary in peptide chemistry and

$R_7$ either has the abovementioned meaning of $R_3$ or represents a protecting group, and

n has the abovementioned meaning,

and, in the case where $R_7$ represents a protecting group, the latter is removed

and, if appropriate, the radicals $R_2$ and $R_4$ are introduced by alkylation.

6. Medicaments, containing benzodiazepines of the formula

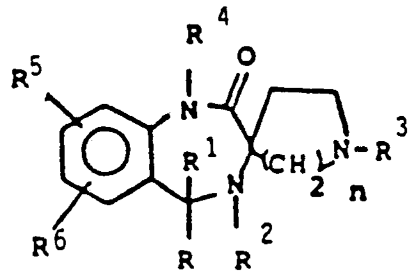

in which

R represents H, straight-chain, branched or cyclic alkyl having 1 to 8 carbon atoms which is saturated or contains one double bond, an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $OCH_3$,

represents a phenyl radical which optionally contains one or two substituents from the series comprising Cl, F, Br, CN, $CF_3$, $NO_2$, $CH_3$, $C_2H_5$ or $OCH_3$, or represents a 5- or 6-membered aromatic radical having 1 or 2 heteroatoms from the series comprising O, N and S,

$R_1$ represents H,

$R_2$, $R_3$ and $R_4$, independently of one another, represent H, an alkyl radical having 1 to 6 carbon atoms, or an aralkyl radical having an alkyl or alkylene chain of 1 to 3 C atoms and a phenyl radical which is unsubstituted or optionally contains 1 or 2 substituents from the series comprising Cl, Br, F, CN, $CF_3$, $NO_2$, $CH_3$, $C_2H_5$ or $CH_3$, and, where appropriate,

$R_1$ and $R_2$ together can also form a bond,

$R_5$ and $R_6$, independently of one another, represents H, F, Cl, Br, CN, $CF_3$, $NO_2$, $CH_3$, $C_2H_5$ or $OCH_3$, and

n represents an integer from 1 to 3.

7. Medicaments according to Claim 6, containing 0.1 to 99.5 % by weight of the benzodiazepine.

8. Use of benzodiazepines of the formula

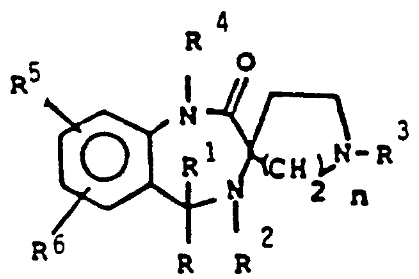

in which

R represents H, straight-chain, branched or cyclic alkyl having 1 to 8 carbon atoms which is saturated or contains one double bond, an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $OCH_3$,

represents a phenyl radical which optionally contains one or two substituents from the series comprising Cl, F, Br, CN, $CF_3$, $NO_2$, $CH_3$, $C_2H_5$ or $OCH_3$, or represents a 5- or 6-membered aromatic radical having 1 or 2 heteroatoms from the series comprising O, N and S,

$R_1$ represents H,

$R_2$, $R_3$ and $R_4$, independently of one another, represent H, an alkyl radical having 1 to 6 carbon atoms, or an aralkyl radical having an alkyl or alkylene chain of 1 to 3 C atoms and a phenyl radical which is unsubstituted or optionally contains one or two substituents from the series comprising Cl, Br, F, CN, $CF_3$, $NO_2$, $CH_3$, $C_2H_5$ or $CH_3$, and, where appropriate,

$R_1$ and $R_2$ together can also form a bond,

$R_5$ and $R_6$, independently of one another, represents H, F, Cl, Br, CN, $CF_3$, $NO_2$, $CH_3$, $C_2H_5$ or $OCH_3$, and

n represents an integer from 1 to 3,

for the preparation of medicaments.

9. Use according to Claim 8 for the preparation of nootropics.

10. Use according to Claim 8 for the preparation of medicaments for treatments of age-induced brain dysfunctions.

11. Use according to Claim 8 for the preparation of medicaments for treatments of amnesia.

**Revendications**

1. Benzodiazépines de formule:

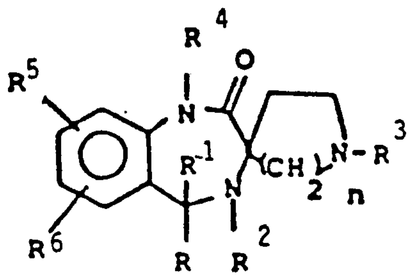

dans laquelle:

R représente H, un groupe alkyle ayant 1 à 8 atomes de carbone, et qui est saturé ou contient une double liaison et est linéaire, ramifié ou cyclique, un reste aralkyle comportant une chaîne alkyle ou alkylène ayant 1 à 3 atomes de carbone et un reste phényle non substitué ou comportant éventuellement un ou deux substituants choisis parmi Cl, Br, F, CN, $CF_3$, $NO_2$, $CH_3$, $C_2H_5$ ou $OCH_3$,

un reste phényle comportant éventuellement un ou deux substituants choisis parmi Cl, F, Br, CN, $CF_3$, $NO_2$, $CH_3$, $C_2H_5$ ou $OCH_3$, ou un reste aromatique pentagonal ou hexagonal comportant 1 ou 2 hétéroatomes choisis parmi O, N, S,

$R_1$ représente H,

$R_2$, $R_3$ et $R_4$ représentent, indépendamment l'un de l'autre, H, un reste alkyle ayant 1 à 6 atomes de carbone, ou un reste aralkyle comportant une chaîne alkyle ou alkylène ayant 1 à 3 atomes de carbone et un reste phényle, non substitué ou comportant éventuellement un ou deux substituants choisis parmi Cl, Br, F, CN, $CF_3$, $NO_2$, $CH_3$, $C_2H_5$ ou $CH_3$, et, éventuellement,

$R_1$ et $R_2$ peuvent également former ensemble une liaison,

$R_5$ et $R_6$ représentent, indépendamment l'un de l'autre, H, F, Cl, Br, CN, $CF_3$, $NO_2$, $CH_3$, $C_2H_5$ ou $OCH_3$, et

n est un nombre entier valant 1 à 3.

2. Benzodiazépines selon la revendication 1, dans lesquelles:

R représente un reste phényle non substitué ou comportant éventuellement un ou deux substituants choisis parmi Cl, F, Br, CN, $CF_3$ ou $NO_2$, ou bien R représente un reste hétéro-aromatique pentagonal ou hexagonal comportant 1 ou 2 hétéroatomes choisis parmi O, N, S,

$R_1$ représente H,

$R_2$, $R_3$ et $R_4$ représentent, indépendamment l'un de l'autre, H ou un reste alkyle ayant 1 à 4 atomes de carbone, et

$R_1$ et $R_2$ peuvent également former ensemble une liaison,

$R_5$ et $R_6$ et représentent, indépendamment l'un de l'autre, H, F, Cl, Br, CN, $CF_3$ ou $NO_2$, et

n vaut 2.

3. Benzodiazépine selon les revendications 1 et 2, répondant à la formule:

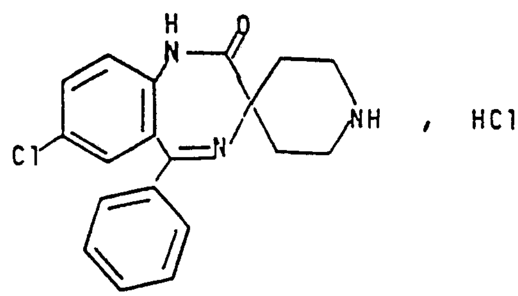

4. Benzodiazépines de formule:

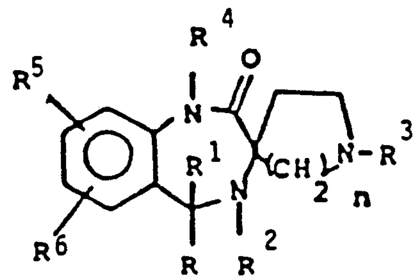

dans laquelle:

R représente H, un reste alkyle ayant 1 à 8 atomes de carbone, saturé ou contenant une double liaison et qui est linéaire, ramifié ou cyclique,

un reste aralkyle comportant une chaîne alkyle ou alkylène ayant 1 à 3 atomes de carbone et un reste phényle non substitué ou comportant éventuellement un ou deux substituants choisis parmi Cl, Br, F, CN, $CF_3$, $NO_2$, $CH_3$, $C_2H_5$ ou $OCH_3$,

un reste phényle comportant éventuellement un ou deux substituants choisis parmi Cl, F, Br, CN, $CF_3$, $NO_2$, $CH_3$, $C_2H_5$ ou $OCH_3$, ou un reste aromatique pentagonal ou hexagonal comportant un ou deux hétéroatomes, choisis parmi O, N, S,

$R_1$ représente H,

$R_2$, $R_3$ et $R_4$ représentent, indépendamment l'un de l'autre, H, un reste alkyle ayant 1 à 6 atomes de carbone, ou un reste aralkyle comportant une chaîne alkyle ou alkylène ayant 1 à 3 atomes de carbone et un reste phényle non substitué ou comportant éventuellement un ou deux substituants choisis parmi Cl, Br, F, CN, $CF_3$, $NO_2$, $CH_3$, $C_2H_5$ ou $CH_3$, et, éventuellement,

$R_1$ et $R_2$ peuvent également former ensemble une liaison,

$R_5$ et $R_6$ représentent, indépendamment l'un de l'autre, H, F, Cl, Br, CN, $CF_3$, $NO_2$, $CH_3$, $C_2H_5$ ou $OCH_3$, et

n est un nombre entier valant 1 à 3,

pour un traitement thérapeutique.

5. Procédé de préparation de benzodiazépines de formule:

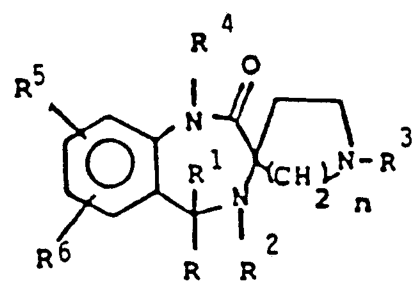

dans laquelle:

R représente H, un groupe alkyle ayant 1 à 8 atomes de carbone, saturé ou contenant une double liaison et qui est linéaire, ramifié ou cyclique,

un reste aralkyle comportant une chaîne alkyle ou alkylène ayant 1 à 3 atomes de carbone et un reste phényle non substitué ou comportant éventuellement un ou deux substituants choisis parmi Cl, Br, F, CN, $CF_3$, $NO_2$, $CH_3$, $C_2H_5$ ou $OCH_3$,

un reste phényle comportant éventuellement un ou deux substituants choisis parmi Cl, F, Br, CN, $CF_3$, $NO_2$, $CH_3$, $C_2H_5$ ou $OCH_3$, ou un reste aromatique pentagonal ou hexagonal comportant 1 ou 2 hétéroatomes choisis parmi O, N, S,

$R_1$ représente H,

$R_2$, $R_3$ et $R_4$ représentent, indépendamment l'un de l'autre, H, un reste alkyle ayant 1 à 6 atomes de carbone, ou un reste aralkyle comportant une chaîne alkyle ou alkylène ayant 1 à 3 atomes de carbone et un reste phényle non substitué, ou portant éventuellement un ou deux substituants choisis parmi Cl, Br, F, CN, $CF_3$, $NO_2$, $CH_3$, $C_2H_5$ ou $CH_3$, et, éventuellement,

$R_1$ et $R_2$ peuvent également former ensemble une liaison,

$R_5$ et $R_6$ représentent, indépendamment l'un de l'autre, H, F, Cl, Br, CN, $CF_3$, $NO_2$, $CH_3$, $C_2H_5$ ou $OCH_3$, et

n est un nombre entier valant 1 à 3,

procédé caractérisé en ce qu'on fait réagir des composés de formule générale:

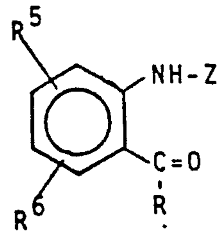

dans laquelle:

$R_5$, $R_6$ et R ont le sens indiqué ci-dessus, et

Z représente H ou un groupe protecteur,

avec des composés de formule générale:

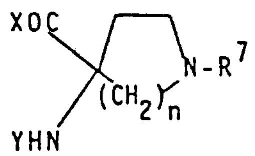

dans laquelle:

COX représente une fonction carboxyle ou une fonction carboxyle activée, telle qu'on l'utilise pour la fixation de liaison amide d'acide,

Y représente H ou un groupe protecteur usuel dans la chimie des peptides, et

$R_7$ a le sens indiqué ci-dessus pour $R_3$ ou représente un groupe protecteur, et

n a le sens indiqué ci-dessus,

et, si $R_7$ est un groupe protecteur, on détache celui-ci, et éventuellement on introduit, par alkylation, les restes $R_2$ et $R_4$.

6. Médicament, contenant des benzodiazépines de formule:

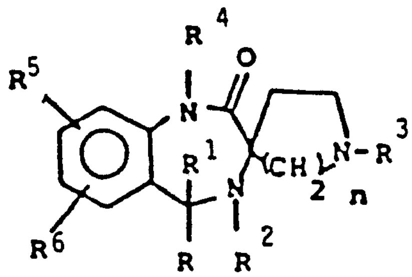

dans laquelle:

R représente H, un groupe alkyle ayant 1 à 8 atomes de carbone, saturé ou contenant une double liaison et qui est linéaire, ramifié ou cyclique,

un reste aralkyle comportant une chaîne alkyle ou alkylène ayant 1 à 3 atomes de carbone et un reste phényle non substitué ou comportant éventuellement un ou deux substituants choisis parmi Cl, Br, F, CN, $CF_3$, $NO_2$, $CH_3$, $C_2H_5$ ou $OCH_3$,

un reste phényle comportant éventuellement un ou deux substituants choisis parmi Cl, F, Br, CN, $CF_3$, $NO_2$, $CH_3$, $C_2H_5$ ou $OCH_3$, ou un reste aromatique pentagonal ou hexagonal comportant 1 ou 2 hétéroatomes choisis parmi O, N, S,

$R_1$ représente H,

$R_2$, $R_3$ et $R_4$ représentent, indépendamment l'un de l'autre, H, un reste alkyle ayant 1 à 6 atomes de carbone, ou un reste aralkyle comportant une chaîne alkyle ou alkylène ayant 1 à 3 atomes de carbone et un reste phényle non substitué, ou comportant éventuellement un ou deux substituants choisis parmi Cl, Br, F, CN, $CF_3$, $NO_2$, $CH_3$, $C_2H_5$ ou $CH_3$, et, éventuellement,

$R_1$ et $R_2$ peuvent également former ensemble une liaison,

$R_5$ et $R_6$ représentent, indépendamment l'un de l'autre, H, F, Cl, Br, CN, $CF_3$, $NO_2$, $CH_3$, $C_2H_5$ ou $OCH_3$, et

n est un nombre entier valant 1 à 3.

7. Médicament selon la revendication 6, contenant 0,1 à 99,5 % en poids de la benzodiazépine.

8. Utilisation de benzodiazépines de formule:

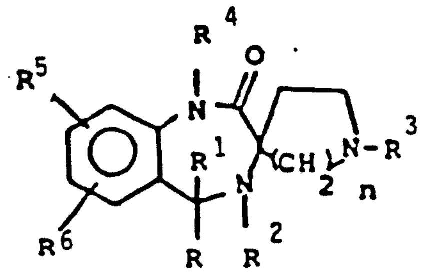

dans laquelle:

R représente H, un groupe alkyle ayant 1 à 8 atomes de carbone, saturé ou contenant une double liaison et qui est linéaire, ramifié ou cyclique,

un reste aralkyle ayant une chaîne alkyle ou alkylène comportant 1 à 3 atomes de carbone et un reste phényle non substitué ou comportant éventuellement un ou deux substituants choisis parmi Cl, Br, F, CN, $CF_3$, $NO_2$, $CH_3$, $C_2H_5$ ou $OCH_3$,

un reste phényle comportant éventuellement un ou deux substituants choisis parmi Cl, F, Br, CN, $CF_3$, $NO_2$, $CH_3$, $C_2H_5$ ou $OCH_3$, ou un reste aromatique pentagonal ou hexagonal comportant 1 ou 2 hétéroatomes choisis parmi O, N, S,

$R_1$ représente H,

$R_2$, $R_3$ et $R_4$ représentent, indépendamment l'un de l'autre, H, un reste alkyle ayant 1 à 6 atomes de carbone, ou un reste aralkyle comportant une chaîne alkyle ou alkylène ayant 1 à 3 atomes de carbone et un reste phényle non substitué, ou comportant éventuellement un ou deux substituants choisis parmi Cl, Br, F, CN, $CF_3$, $NO_2$, $CH_3$, $C_2H_5$ ou $CH_3$, et, éventuellement,

$R_1$ et $R_2$ peuvent également former ensemble une liaison,

$R_5$ et $R_6$ représentent, indépendamment l'un de l'autre, H, F, Cl, Br, CN, $CF_3$, $NO_2$, $CH_3$, $C_2H_5$ ou $OCH_3$, et

n est un nombre entier valant 1 à 3,

pour préparer des médicaments.

9. Utilisation selon la revendication 8 pour préparer des nootropes.

10. Utilisation selon la revendication 8 pour préparer des médicaments destinés à traiter des troubles, provoqués par le vieillissement, du fonctionnement cérébral.

11. Utilisation selon la revendication 8, pour préparer des médicaments destinés à traiter des amnésies.